# EUROPÄISCHE PATENTSCHRIFT

(11) **EP 2 379 508 B1**
(45) Veröffentlichungstag und Bekanntmachung des Hinweises auf die Patenterteilung: **04.06.2014**
(21) Anmeldenummer: 09768345.2
(22) Anmeldetag: 12.12.2009
(51) Int. Cl.: C07D 237/16, A01N 43/58

(54) **HERBIZID UND INSEKTIZID WIRKSAME 4-PHENYLSUBSTITUIERTE PYRIDAZINONE**
Herbicidal and insecticidal phenyl-substituted pyridazinones
Pyridazinones substitués par phényle agissant en tant qu'herbicide et insecticide

(30) Priorität: 19.12.2008 EP 08022104
(43) Veröffentlichungstag der Anmeldung: 26.10.2011
(73) Patentinhaber: Bayer CropScience AG, 40789 Monheim (DE)
(72) Erfinder: LEHR, Stefan, 65835 Liederbach (DE); SCHENKE, Thomas, 51469 Bergisch Gladbach (DE); ROSINGER, Christopher Hugh, 65719 Hofheim (DE); FISCHER, Reiner, 40789 Monheim (DE); HÄUSER-HAHN, Isolde, 51375 Leverkusen (DE); FEUCHT, Dieter, 65760 Eschborn (DE); DITTGEN, Jan, 60316 Frankfurt (DE); CRISTAU, Pierre, 69009 Lyon (FR); GAERTZEN, Oliver, 50931 Köln (DE); HERRMANN, Stefan, 40764 Langenfeld (DE); MALSAM, Olga, 51503 Rösrath (DE); FRANKEN, Eva-Maria, 51381 Leverkusen (DE)
(86) Internationale Anmeldenummer: PCT/EP2009/008908
(87) Internationale Veröffentlichungsnummer: WO 2010/069525

(56) Entgegenhaltungen:
- WO-A-2007/119434
- JP-A- 11 152 273
- STEVENSON THOMAS M ET AL: "Application of cross-coupling and metalation chemistry of 3(2H)-pyridazinones to fungicide and herbicide discovery" JOURNAL OF HETEROCYCLIC CHEMISTRY, HETEROCORPORATION. PROVO, US, Bd. 42, Nr. 3, 1. April 2005 (2005-04-01), Seiten 427-435, XP002442886 ISSN: 0022-152X

## Beschreibung

Die Erfindung betrifft das technische Gebiet der Pflanzenschutzmittel, insbesondere das der Herbizide zur selektiven Bekämpfung von Unkräutern und Ungräsern in Nutzpflanzenkulturen.

Speziell betrifft sie 4-phenylsubstituierte Pyridazinone, Verfahren zu ihrer Herstellung und ihre Verwendung als Herbizide und Insektizide.

Verschiedene Schriften beschreiben substituierte 4-Phenylpyridazinone mit herbiziden Eigenschaften. Aus Stevenson et. al, J. Het. Chem., (2005), 427 ff. sind 2-Methyl-4-phenylpyridazinone bekannt. In W02007/119434 A1 werden 4-Phenylpyridazinone genannt, die in 2-Position des Phenylrings einen Alkylrest tragen. WO2009/035150 A2 offenbart 4-Phenylpyridazinone genannt, die in 2-Position des Phenylrings einen Alkyl- oder Alkoxyrest tragen, und an den anderen Positionen gegebenenfalls durch Halogenatome oder andere Reste substituiert sind.

Die aus diesen Schriften bekannten Verbindungen zeigen jedoch häufig eine nicht ausreichende herbizide Wirksamkeit. Aufgabe der vorliegenden Erfindung ist daher die Bereitstellung alternativer herbizid wirksamer Verbindungen.

Es wurde gefunden, daß 4-Phenylpyridazinone, deren Phenylring bestimmte Substituenten trägt, als Herbizide besonders gut geeignet sind.

Ein Gegenstand der vorliegenden Erfindung sind 4-Phenylpyridazinone der Formel (I) oder deren Salze 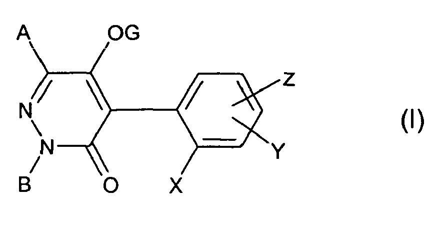 worin
A bedeutet unabhängig voneinander jeweils Wasserstoff, (C₃-C₆)-Cycloalkyl oder durch n Reste aus der Gruppe bestehend aus Halogen, (C₃-C₆)-Cycloalkyl, Phenyl und Halogenphenyl substituiertes (C₁-C₆)-Alkyl,
B bedeutet unabhängig voneinander (C₃-C₆)-Cycloalkyl oder durch m Reste aus der Gruppe bestehend aus Halogen, (C₃-C₆)-Cycloalkyl, Phenyl und Halogenphenyl substituiertes (C₁-C₆)-Alkyl;
m bedeutet 0, 1, 2 oder 3;
n bedeutet 0, 1, 2 oder 3, mit der Maßgabe, dass m und n nicht gleichzeitig 0 bedeuten;
G bedeutet Wasserstoff, C(=O)R¹, C(=L)MR², SO₂R³, P(=L)R⁴R⁵, C(=L)NR⁶R⁷, E;
E bedeutet ein Metallionäquivalent oder ein Ammoniumion;
L bedeutet Sauerstoff oder Schwefel;
M bedeutet Sauerstoff oder Schwefel;
R¹ bedeutet jeweils durch n Halogenatome substituiertes (C₁-C₆)-Alkyl, (C₂-C₆)-Alkenyl, (C₁-C₄)-Alkoxy-(C₁-C₆)-alkyl, Di-(C₁-C₄)-alkoxy-(C₁-C₆)-alkyl oder (C₁-C₄)-Alkylthio-(C₁-C₆)-alkyl,
   einen durch n Reste aus der Gruppe bestehend aus Halogen, (C₁-C₄)-Alkyl und (C₁-C₄)-Alkoxy substituierten vollständig gesättigten 3- bis 6-gliedrigen Ring bestehend aus 3 bis 5 Kohlenstoffatomen und 1 bis 3 Heteroatomen aus der Gruppe Sauerstoff, Schwefel und Stickstoff,
   durch n Reste aus der Gruppe bestehend aus Halogen, (C₁-C₄)-Alkyl und (C₁-C₄)-Alkoxy substituiertes (C₃-C₆)-Cycloalkyl, Phenyl, Phenyl-(C₁-C₄)-alkyl, Heteroaryl, Phenoxy-(C₁-C₄)-alkyl oder Heteroaryloxy-(C₁-C₄)-alkyl;
R² bedeutet jeweils durch n Halogenatome substituiertes (C₁-C₆)-Alkyl, (C₂-C₆)-Alkenyl, (C₁-C₄)-Alkoxy-(C₁-C₆)-alkyl oder Di-(C₁-C₄)-alkoxy-(C₁-C₆)-alkyl, oder durch jeweils n Reste aus der Gruppe bestehend aus Halogen, (C₁-C₄)-Alkyl und (C₁-C₄)-Alkoxy substituiertes (C₃-C₆)-Cycloalkyl, Phenyl oder Benzyl;
R³, R⁴ und R⁵ bedeuten unabhängig voneinander jeweils durch n Halogenatome substituiertes (C₁-C₆)-Alkyl, (C₁-C₄)-Alkoxy, N-(C₁-C₆)-Alkylamino, N,N-Di-(C₁-C₆)-Alkylamino, (C₁-C₄)-Alkylthio, (C₂-C₄)-Alkenyl oder (C₃-C₆)-Cycloalkylthio,
   oder durch n Reste aus der Gruppe bestehend aus Halogen, (C₁-C₄)-Alkyl und (C₁-C₄)-Alkoxy substituiertes Phenyl, Benzyl, Phenoxy oder Phenylthio;
R⁶ und R⁷ bedeuten unabhängig voneinander jeweils Wasserstoff,
   durch n Halogenatome substituiertes (C₁-C₆)-Alkyl, (C₃-C₆)-Cycloalkyl, (C₂-C₆)-Alkenyl, (C₁-C₆)-Alkoxy oder (C₁-C₄)-Alkoxy-(C₁-C₆)-alkyl,
   durch jeweils n Reste aus der Gruppe bestehend aus Halogen, (C₁-C₄)-Alkyl und (C₁-C₄)-Alkoxy substituiertes Phenyl oder Benzyl;
   oder R⁶ und R⁷ bilden gemeinsam mit dem N-Atom, an das sie gebunden sind, einen 3- bis 6-gliedrigen Ring enthaltend 2 bis 5 Kohlenstoffatomen und 0 oder 1 Sauerstoff- oder Schwefelatome;
und
a)
   - X: bedeutet Wasserstoff, (C₁-C₆)-Alkyl oder (C₃-C₆)-Cycloalkyl;
   - Y: bedeutet Halogen, Cyano, Nitro, Halogen-(C₁-C₆)-alkyl, Halogen-(C₁-C₆)-alkoxy, (C₃-C₆)-Cycloalkyl, (C₁-C₆)-Alkoxy oder durch n Halogenatome substituiertes Phenyl,
   - Z: bedeutet Wasserstoff, Halogen, Cyano, Nitro, (C₁-C₆)-Alkyl, Halogen-(C₁-C₆)-alkyl, Halogen-(C₁-C₆)-alkoxy, (C₃-C₆)-Cycloalkyl oder durch n Halogenatome substituiertes Phenyl;
   oder
b)
   - X: bedeutet Halogen, Cyano, Nitro oder jeweils durch n Halogenatome substituiertes (C₁-C₆)-Alkyl, (C₃-C₆)-Cycloalkyl, (C₁-C₆)-Alkoxy oder Phenyl;
   - Y und Z: bedeuten unabhängig voneinander jeweils Wasserstoff, Halogen, Cyano, Nitro, (C₃-C₆)-Cycloalkyl oder durch jeweils n Halogenatome substituiertes (C₁-C₆)-Alkyl, (C₁-C₆)-Alkoxy oder Phenyl.

Die erfindungsgemäßen Verbindungen der Formel (I) können für den Fall, dass G und/oder B für Wasserstoff stehen, in Abhängigkeit von äußeren Bedingungen, wie pH-Wert, Lösungsmittel und Temperatur in verschiedenen tautomeren Strukturen vorliegen, die alle von der allgemeinen Formel (I) umfasst sein sollen:

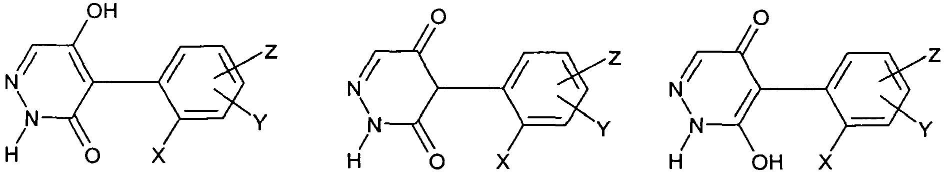

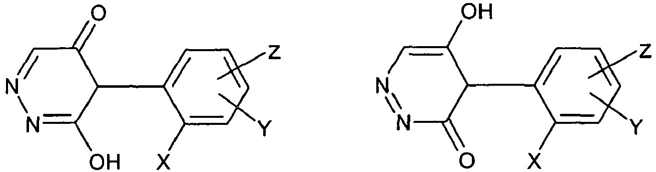

In allen nachfolgenden Strukturen haben die Substituenten, sofern nicht gesondert definiert, dieselbe Bedeutung wie oben zu Verbindungen der Formel (I) angegeben.

Erfindungsgemäße Verbindungen der Formel (I), worin G für Wasserstoff steht, können beispielsweise gemäß der in Schema 1 angegebenen Methode durch baseninduzierte Kondensationsreaktion von Verbindungen der Formel (II) hergestellt werden. Darin steht R⁹ für (C₁-C₆)-Alkyl, insbesonders für Methyl oder Ethyl.

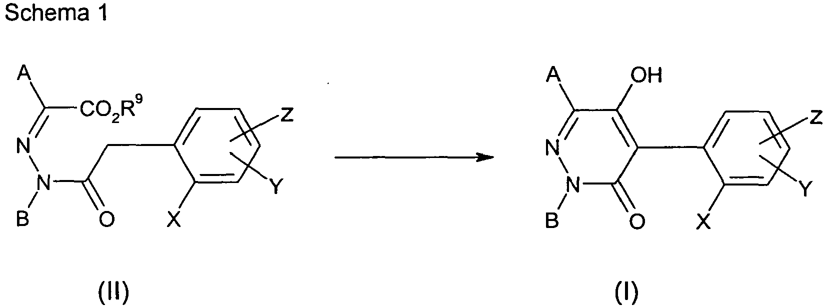

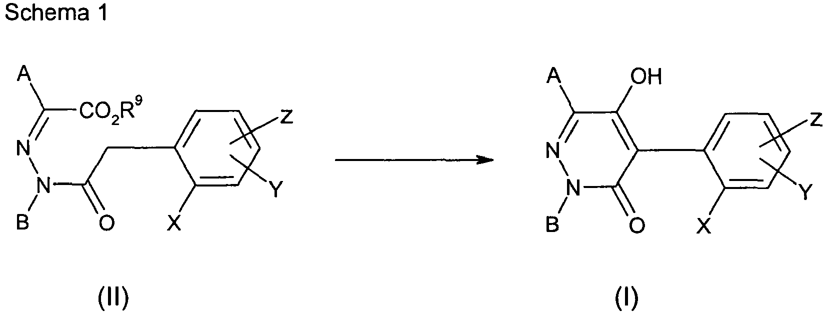

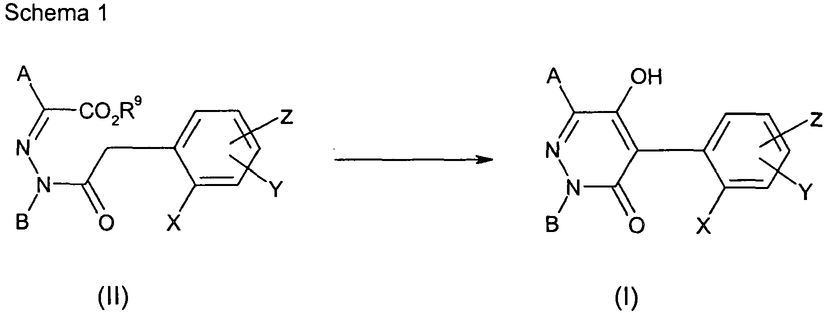

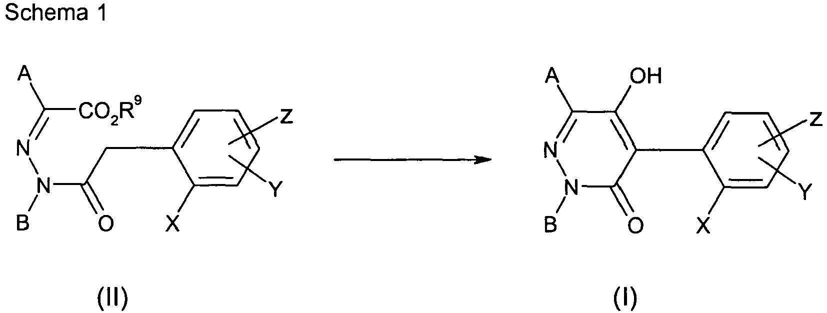

Verbindungen der Formel (II) sind neu und ebenfalls ein Gegenstand vorliegender Erfindung. Sie können beispielsweise gemäß der in Schema 1 a angegebenen Methode durch Reaktion von Hydrazonocarbonsäurederivaten mit Phenylessigsäurederivaten hergestellt werden. Darin steht U für eine durch Carbonsäureaktivierungsreagenzien, wie Carbonyldiimidazol, Carbonyldiimide (wie z.B. Dicyclohexylcarbondiimid), Phosphorylierungsreagenzien (wie z.B. POCl₃, BOP-CI), Halogenierungsmittel wie z.B. Thionylchlorid, Oxalylchlorid, Phosgen oder Chlorameisensäureester eingeführte Abgangsgruppe. Solche Methoden sind auch aus WO2007/119434 und dort zitierten Dokumenten dem Fachmann bekannt.

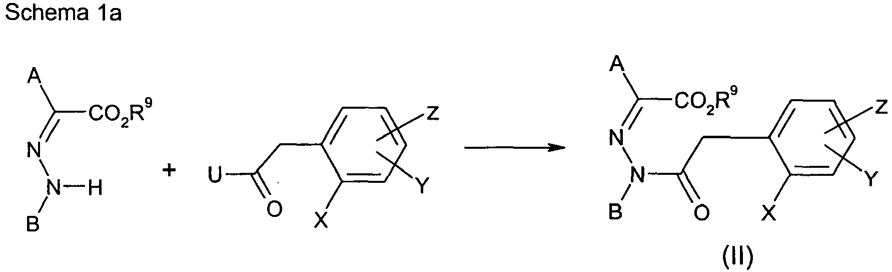

Verbindungen der Formel (II) können beispielsweise auch gemäß der in Schema 1b angegebenen Methode durch dem Fachmann aus Zh. Obs. Khim. 1992, 62, 2262 bekannte Reaktion von Hydraziden (IIa) mit Ketocarbonsäuren der Formel A-CO-CO₂R⁹ hergestellt werden.

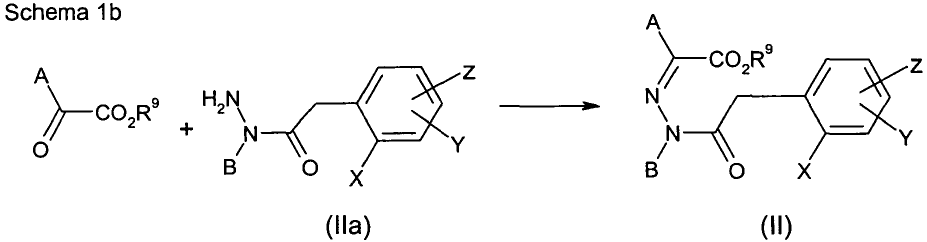

Die in Schema 1 b genannten Hydrazide der Formel (IIa) sind neu und ebenfalls ein Gegenstand vorliegender Erfindung. Sie können beispielsweise gemäß durch Reaktion von Hydrazinen der Formel B-NH-NH₂ mit den in Schema 1a genannten Phenylessigsäurederivaten gemäß der in J. Org. Chem. 1980, 45, 3673 beschriebenen Methode hergestellt werden. Die in Schema 1a genannten Hydrazide können aus den in Schema 1 b genannten und an sich bekannten Ketocarbonsäuren A-CO-CO₂R⁹ bespielsweise gemäß der in J. Med. Chem. 1985 (28), 1436 beschriebenen Methoden hergestellt werden.

Die zur Herstellung der in Schema 1a genannten Phenylessigsäurederivaten notwendigen freien Phenylessigsäuren, d.h. solche worin U für Hydroxy steht, sind bekannt oder lassen sich nach an sich und beispielsweise aus WO 2005/075401, WO 2001/96277, WO 1996/35664 und WO 1996/25395 bekannten Verfahren herstellen.

Bestimmte Phenylessigsäurederivate lassen sich aber auch unter Verwendung von Essigesterenolaten in Gegenwart von Palladiumkatalysatoren, z.B. gebildet aus einer Palladiumquelle (z.B. Pd₂(dba)₃ oder Pd(OAc)₂) und einem Liganden (z.B. (t-Bu)₃P, iMes*HCl oder 2'-(N,N-Dimethylamino)-2-(dicyclohexylphosphanyl)biphenyl) hergestellt (WO 2005/048710, J. Am. Chem. Soc 2002. 124, 12557, J. Am. Chem. Soc 2003. 125, 11176 oder J. Am. Chem. Soc. 2001, 123, 799) herstellen. Darüber hinaus lassen sich bestimmte substituierte Arylhalogenide unter Kupferkatalyse in die korrespondierenden substituierten Malonester überführen (z.B. beschrieben in Org. Lett. 2002, 2, 269, WO 2004/108727), welche nach bekannten Methoden in Phenylessigsäuren überführt werden können.

Erfindungsgemäße Verbindungen der Formel (I), worin G für Wasserstoff steht, können beispielsweise auch gemäß der in Schema 2 angegebenen Methode durch Reaktion von Verbindungen der Formel (I), worin G für R⁸ steht, mit starken Mineralbasen, wie Natrium- oder Kaliumhydroxid, oder in konzentrierten Mineralsäuren, wie Bromwassserstoffsäure, hergestellt werden.

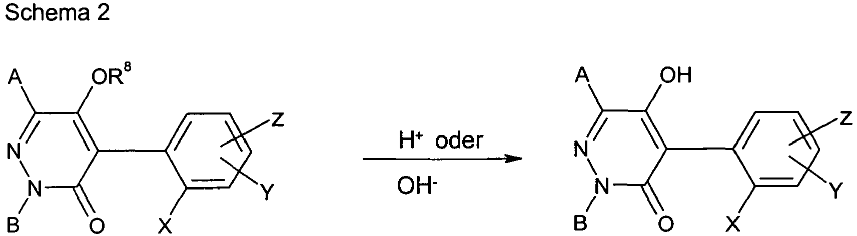

Erfindungsgemäße Verbindungen der Formel (I), worin G für C(=O)R¹ steht, können beispielsweise durch dem Fachmann bekannte Reaktionen von Verbindungen der Formel (I), worin G für Wasserstoff steht, mit Carbonsäurehalogeniden der Formel Hal-CO-R¹ oder mit Carbonsäureanhydriden der Formel R¹-CO-O-CO-R¹ hergestellt werden.

Erfindungsgemäße Verbindungen der Formel (I), worin G für C(=L)MR² steht, können beispielsweise durch dem Fachmann bekannte Reaktionen von Verbindungen der Formel (I), worin G für Wasserstoff steht, mit a) Chlorameisensäureestern oder Chlorameisensäurethioestern der Formel R²-M-COOR¹ oder b) mit Chlorameisensäurehalogeniden oder Chlorameisensäurethiohalogeniden hergestellt werden.

Erfindungsgemäße Verbindungen der Formel (I), worin G für SO₂R³ steht, können beispielsweise durch dem Fachmann bekannte Reaktionen von Verbindungen der Formel (I), worin G für Wasserstoff steht, mit Sulfonsäurechloriden der Formel R³-SO₂-Cl hergestellt werden.

Erfindungsgemäße Verbindungen der Formel (I), worin G für P(=L)R⁴R⁵ steht, können beispielsweise durch dem Fachmann bekannte Reaktionen von Verbindungen der Formel (I), worin G für Wasserstoff steht, mit Phosphorsäurechloriden der Formel Hal-P(=L)R⁴R⁵ hergestellt werden.

Erfindungsgemäße Verbindungen der Formel (I), worin G für E steht, können beispielsweise durch dem Fachmann bekannte Reaktionen von Verbindungen der Formel (I), worin G für Wasserstoff steht, mit Metallverbindungen der Formel Me(OR¹⁰)ₜ oder mit Aminen hergestellt werden. Darin bedeutet Me ein ein- oder zweiwertiges Metallion, bevorzugt ein Alkali- oder Erdalkalimetall wie Lithium, Natrium, Kalium, Magnesium oder Calcium. Der Index t steht für 1 oder 2. Ein Ammoniumion bedeutet die Gruppe NH₄⁺ oder R¹³R¹⁴R¹⁵R¹⁶N⁺, worin R¹³, R¹⁴, R¹⁵ und R¹⁶ unabhängig voneinander vorzugsweise (C₁-C₆)-Alkyl oder Benzyl bedeuten.

Erfindungsgemäße Verbindungen der Formel (I), worin G für C(=L)NR⁶R⁷ steht, können beispielsweise durch dem Fachmann bekannte Reaktionen von Verbindungen der Formel (I), worin G für Wasserstoff steht, mit Isocyanaten oder Isothiocyanaten der Formel R⁶-N=C=L oder mit Carbamidsäurechloriden oder Thiocarbamidsäurechloriden der Formel R⁶R⁷N-C(=L)Cl hergestellt werden.

Erfindungsgemäße Verbindungen der Formel (I), worin G für R⁸ steht, können beispielsweise durch dem Fachmann bekannte Reaktionen von Verbindungen der Formel (I), worin G für Wasserstoff steht, mit Verbindungen der Formel R⁸-W, worin W für Chlor, Brom, lod oder R⁹-SO₂-O steht, hergestellt werden. R⁹ bedeutet C₁-C₆-Alkyl, Phenyl, Tolyl oder Halogen-C₁-C₆-Alkyl, vorzugsweise Methyl, Trifluormethyl oder Tolyl.

Erfindungsgemäße Verbindungen der Formel (I), worin G für R⁸ steht, können beispielsweise auch gemäß Schema 3 durch dem Fachmann bekannte Reaktionen von Verbindungen der Formel (III) mit Verbindungen der Formel (IV) hergestellt werden. Darin steht Z' für Brom oder lod, und Q bedeutet eine Trialkylzinngruppe, eine Magnesiumhalogenidgruppe oder bevorzugt eine Boronsäure oder deren Ester. Diese Reaktionen werden üblicherweise in Gegenwart eines Katalysators (z. B. Pd-Salze oder Pd-Komplexe) und in Gegenwart einer Base (z.B. Natriumcarbonat, Kaliumphosphat) durchgeführt.

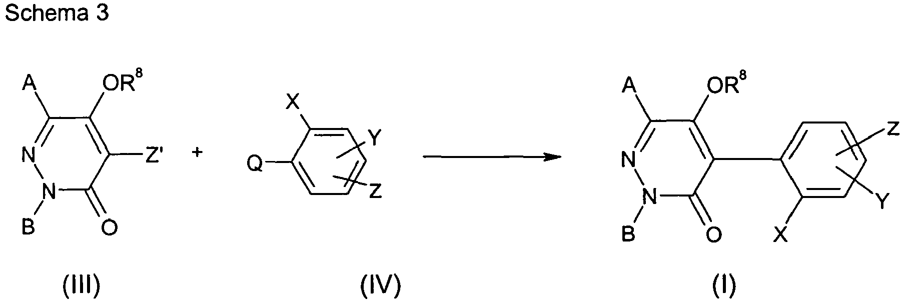

Je nach Art der oben definierten Substituenten weisen die Verbindungen der Formel (I) saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel (I) Amino, Alkylamino oder andere, basische Eigenschaften induzierende Gruppen, so können diese Verbindungen mit Säuren zu Salzen umgesetzt werden oder fallen durch die Synthese direkt als Salze an.
Beispiele für anorganische Säuren sind Halogenwasserstoffsäuren wie Fluorwasserstoff, Chlorwasserstoff, Bromwasserstoff und lodwasserstoff, Schwefelsäure, Phosphorsäure und Salpetersäure und saure Salze wie NaHSO₄ und KHSO₄.
Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, Alkylsulfonsäuren (Sulfonsäuren mit geradkettigen oder verzweigten Alkylresten mit 1 bis 20 Kohlenstoffatomen), Arylsulfonsäuren oder-disulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkylresten mit 1 bis 20 Kohlenstoffatomen), Arylphosphonsäuren oder - diphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.
Tragen die Verbindungen der Formel (I) Hydroxy, Carboxy oder andere, saure Eigenschaften induzierende Gruppen, so können diese Verbindungen mit Basen zu Salzen umgesetzt werden.
Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkyl-Gruppen, Mono-, Di- und Trialkanolamine von (C₁-C₄)-Alkanolen, Cholin sowie Chlorcholin.

Halogen bedeutet Fluor, Chlor, Brom und Jod.

Ein Metallionäquivalent bedeutet ein Metallion mit einer positiven Ladung wie Na⁺, K⁺, (Mg²⁺)_{1/2}, (Ca²⁺)_{1/2}, MgH⁺, CaH⁺, (Al³⁺)_{1/3} (Fe²⁺)_{1/2} oder (Fe³⁺)_{1/3}.

Alkyl bedeutet gesättigte, geradkettige oder verzweigte Kohlenwasserstoffreste mit 1 bis 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methyl-propyl.

Halogenalkyl bedeutet geradkettige oder verzweigte Alkylgruppen mit 1 bis 8 Kohlenstoffatomen (wie oben genannt), wobei in diesen Gruppen teilweise oder vollständig die Wasserstoffatome durch Halogenatome ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl.

Alkenyl bedeutet ungesättigte, geradkettige oder verzweigte Kohlenwasserstoffreste mit 2 bis 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1 ,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Alkoxy bedeutet gesättigte, geradkettige oder verzweigte Alkoxyreste mit 1 bis 8 Kohlenstoffatomen, z.B. C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy; Halogenalkoxy bedeutet geradkettige oder verzweigte Alkoxygruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluor-ethoxy und 1,1,1-Trifluorprop-2-oxy.

Alkylthio bedeutet gesättigte, geradkettige oder verzweigte Alkylthioreste mit 1 bis 8 Kohlenstoffatomen, z.B. C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Di-methylpropylthio,1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und 1-Ethyl-2-methylpropylthio;
Halogenalkylthio bedeutet geradkettige oder verzweigte Alkylthiogruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio.

Heteroaryl bedeutet 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, 1,2,4-Triazol-1-yl, 1,2,4-Triazol-3-yl, 1,2,4-Triazol-4-yl, 1,2,4-Triazol-5-yl, 1,2,3-Triazol-1-yl, 1,2,3-Triazol-2-yl, 1,2,3-Triazol-4-yl, Tetrazol-1-yl, Tetrazol-2-yl, Tetrazol-5-yl, Indol-1-yl, Indol-2-yl, Indol-3-yl, Isoindol-1-yl, Isoindol-2-yl, Benzofur-2-yl, Benzothiophen-2-yl, Benzofur-3-yl, Benzothiophen-3-yl, Benzoxazol-2-yl, Benzothiazol-2-yl, Benzimidazol-2-yl, Indazol-1-yl, Indazol-2-yl, Indazol-3-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl oder 1,2,4-Triazin-6-yl. Dieses Heteroaryl ist jeweils unsubstituiert oder jeweils einfach oder mehrfach gleich oder verschieden substituiert durch Reste ausgewählt aus Fluor, Chlor, Brom, Iod, Cyano, Hydroxy, Mercapto, Amino, Methyl, Ethyl, n-Propyl, Isopropyl, n-Butyl, Isobutyl, sek-Butyl, tert-Butyl, Cyclopropyl, 1-Chlorcyclopropyl, Vinyl, Ethinyl, Methoxy, Ethoxy, Isopropoxy, Methylthio, Ethylthio, Trifluormethylthio, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2,2-Trifluorethyl, Trifluormethoxy, Trifluormethylthio, 2,2,2-Trifluorethoxy, 2,2-Dichlor-2-fluorethyl, 2,2-Difluor-2-chlorethyl, 2-Chlor-2-fluorethyl, 2,2,2-Trichlorethyl, 2,2,2-Trifluorethyl, 2-Fluorethyl, 2,2-Difluorethyl, 2-Methoxyethoxy, Acetyl, Propionyl, Methoxycarbonyl, Ethoxycarbonyl, N-Methylamino, N,N-Dimethylamino, N-Ethylamino, N,N-Diethylamino, Aminocarbonyl, Methylaminocarbonyl, Dimethylaminocarbonyl, Dimethylcarbamoylamino, Methoxycarbonylamino, Methoxycarbonyloxy, Ethoxycarbonylamino, Ethoxycarbonyloxy, Methylsulfamoyl, Dimethylsulfamoyl, Phenyl oder Phenoxy.

Die Verbindungen der Formel (I) können, auch in Abhängigkeit von der Art der Substituenten, als geometrische und/oder optische Isomere oder Isomeren-gemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im Folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Ist eine Gruppe mehrfach durch Reste substituiert, so ist darunter zu verstehen, daß diese Gruppe durch ein oder mehrere gleiche oder verschiedene der genannten Reste substituiert ist.

Bevorzugt sind Verbindungen der allgemeinen Formeln (I-a), (I-b), (I-c), (I-d), (I-e), (I-f) und (I-g):

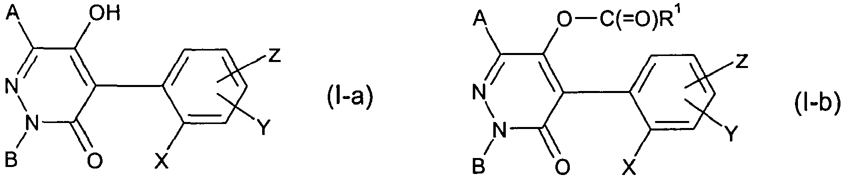

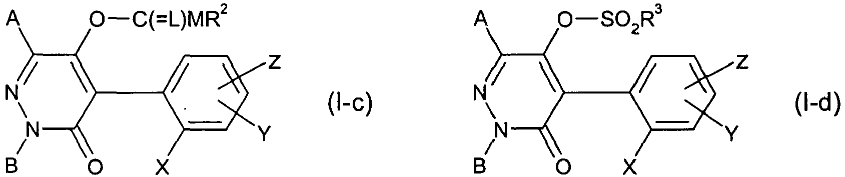

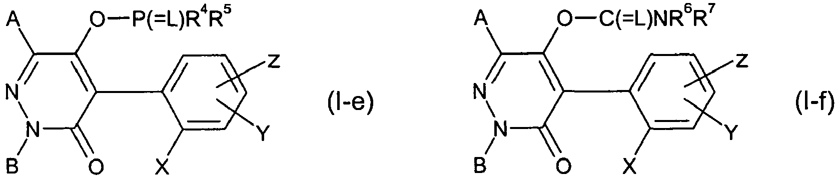

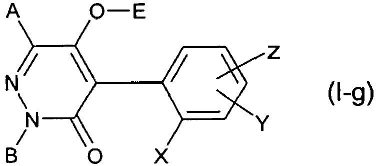

Bevorzugt sind auch Verbindungen der allgemeinen Formel (I), worin
A bedeutet (C₃-C₆)-Cycloalkyl, (C₃-C₆)-Cycloalkyl-(C₁-C₆)-alkyl, Benzyl, Halogenphenyl-(C₁-C₆)-alkyl oder durch n Halogenatome substituiertes (C₁-C₆)-Alkyl;
B bedeutet (C₃-C₆)-Cycloalkyl, (C₃-C₆)-Cycloalkyl-(C₁-C₆)-alkyl, Benzyl, Halogenphenyl-(C₁-C₆)-alkyl oder durch n Halogenatome substituiertes (C₁-C₆)-Alkyl;
m bedeutet 0, 1, 2 oder 3;
n bedeutet 0, 1, 2 oder 3, mit der Maßgabe, dass m und n nicht gleichzeitig 0 bedeuten;
G bedeutet Wasserstoff, C(=O)R¹, C(=L)MR², SO₂R³, P(=L)R⁴R⁵, C(=L)NR⁶R⁷, oder E;
E bedeutet Na⁺, K⁺, (Mg²⁺)_{1/2}, (Ca²⁺)_{1/2} , R¹³R¹⁴R¹⁵R¹⁵N⁺ oder NH₄⁺;
R¹³, R¹⁴, R¹⁵ und R¹⁶ bedeuten unabhängig voneinander (C₁-C₆)-Alkyl oder Benzyl;
L bedeutet Sauerstoff;
M bedeutet Sauerstoff;
R¹ bedeutet durch n Halogenatome substituiertes (C₁-C₆)-Alkyl oder jeweils durch n Reste aus der Gruppe bestehend aus Halogen, (C₁-C₄)-Alkyl und (C₁-C₄)-Alkoxy substituiertes (C₃-C₆)-Cycloalkyl, Phenyl oder Phenyl-(C₁-C₄)-alkyl;
R² bedeutet durch n Halogenatome substituiertes (C₁-C₆)-Alkyl oder jeweils durch n Reste aus der Gruppe bestehend aus Halogen, (C₁-C₄)-Alkyl und (C₁-C₄)-Alkoxy substituiertes (C₃-C₆)-Cycloalkyl, Phenyl oder Benzyl;
R³, R⁴ und R⁵ bedeuten unabhängig voneinander jeweils durch n Halogenatome substituiertes (C₁-C₆)-Alkyl oder durch n Reste aus der Gruppe bestehend aus Halogen, (C₁-C₄)-Alkyl und (C₁-C₄)-Alkoxy substituiertes Phenyl oder Benzyl;
R⁶ und R⁷ bedeuten unabhängig voneinander jeweils Wasserstoff, durch n Halogenatome substituiertes (C₁-C₆)-Alkyl oder durch n Reste aus der Gruppe bestehend aus Halogen, (C₁-C₄)-Alkyl und (C₁-C₄)-Alkoxy substituiertes Phenyl oder Benzyl;
und
a)
   - X: bedeutet Wasserstoff, Methyl, Ethyl oder cyclo-Propyl;
   - Y: bedeutet Halogen, Cyano, Nitro, Halogen-(C₁-C₆)-alkyl, Halogen-(C₁-C₆)-alkoxy, (C₃-C₆)-Cycloalkyl, (C₁-C₆)-Alkoxy oder durch n Halogenatome substituiertes Phenyl,
   - Z: bedeutet Wasserstoff, Halogen, Halogen-(C₁-C₆)-alkyl, Halogen-(C₁-C₆)-alkoxy, (C₃-C₆)-Cycloalkyl oder durch n Halogenatome substituiertes Phenyl,
   oder
b)
   - X: bedeutet Halogen, Cyano, Nitro, Halogen-(C₁-C₆)-alkyl, Halogen-(C₁-C₆)-alkoxy, (C₁-C₆)-Alkoxy oder (C₃-C₆)-Cycloalkyl;
   - Y: bedeutet Wasserstoff, Halogen, Cyano, Nitro, (C₃-C₆)-Cycloalkyl, durch jeweils n Halogenatome substituiertes (C₁-C₆)-Alkyl, (C₁-C₆)-Alkoxy oder Phenyl;
   - Z: bedeutet Wasserstoff, Halogen, Cyano, Nitro, (C₃-C₆)-Cycloalkyl oder durch jeweils n Halogenatome substituiertes (C₁-C₆)-Alkyl, (C₁-C₆)-Alkoxy oder Phenyl.

Besonders bevorzugt sind Verbindungen der allgemeinen Formel (I), worin
A bedeutet cyclo-Propyl, cyclo-Propylmethyl, Benzyl, 2-Chlorphenylmethyl, 3-Chlorphenylmethyl oder 4-Chlorphenylmethyl;
B bedeutet Wasserstoff, 2,2-Difluoroethyl, 2,2,2-trifluoroethyl, cyclo-Propyl, cyclo-Propylmethyl, Benzyl, 2-Chlor-Phenylmethyl, 3-Chlor-Phenylmethyl oder 4-Chlor-Phenylmethyl;
G bedeutet Wasserstoff, C(=O)R¹, C(=L)MR², SO₂R³, P(=L)R⁴R⁵, C(=L)NR⁶R⁷, E oder R⁸;
E bedeutet Na⁺, K⁺, (Mg²⁺)_{1/2}, (Ca²⁺)_{1/2}, (CH₃)₄N⁺ oder NH₄⁺;
L bedeutet Sauerstoff;
M bedeutet Sauerstoff;
R¹ bedeutet (C₁-C₆)-Alkyl oder (C₃-C₆)-Cycloalkyl;
R² bedeutet (C₁-C₆)-Alkyl, (C₃-C₆)-Cycloalkyl oder Benzyl;
R³, R⁴ und R⁵ bedeuten unabhängig voneinander jeweils (C₁-C₆)-Alkyl, Phenyl oder Benzyl;
R⁶ und R⁷ bedeuten unabhängig voneinander jeweils Wasserstoff, (C₁-C₆)-Alkyl, Phenyl oder Benzyl;
und
a)
   - X: bedeutet Wasserstoff, Methyl oder Ethyl;
   - Y: bedeutet Fluor, Brom, Chlor, Iod, Cyano, Nitro, cyclo-Propyl, Methoxy, Ethoxy, Trifluormethyl oder Trifluormethoxy, Phenyl oder Halogenphenyl;
   - Z: bedeutet Wasserstoff, Fluor, Brom, Chlor, Iod, Methyl, Ethyl, Methoxy, Ethoxy, Trifluormethyl oder cyclo-Propyl;
   oder
b)
   - X: bedeutet Fluor, Brom, Chlor, Iod, Cyano, Nitro, Methoxy, Ethoxy, Trifluormethyl, Trifluormethoxy oder cyclo-Propyl
   - Y: bedeutet Wasserstoff, Fluor, Brom, Chlor, Iod, Methyl, Ethyl, Methoxy, Ethoxy, Trifluormethyl, Trifluormethoxy oder cyclo-Propyl;
   - Z: bedeutet Wasserstoff, Fluor, Brom, Chlor, Iod, Methyl, Ethyl, Methoxy, Ethoxy, Trifluormethyl, Trifluormethoxy, cyclo-Propyl, Chlorphenyl oder Fluorphenyl.

Ganz besonders bevorzugt sind die in den Tabellen 1 bis 94 angegebenen Verbindungen der allgemeinen Formel (I), die analog der hier genannten Methoden erhältlich sind.

Die verwendeten Abkürzungen bedeuten:

| | | | | | |
|---|---|---|---|---|---|
| Bz | = Benzyl | c-Pr | = cyclo-Propyl | Et | = Ethyl |
| i-Bu | = iso-Butyl | t-Bu | = tertiär-Butyl | i-Pr | = iso-Propyl |
| Me | = Methyl | Ph | = Phenyl | c | = cyclo |

**Tabelle 1: Erfindungsgemäße Verbindungen der allgemeinen Formel (I), worin G für Wasserstoff steht, A für Methyl steht, und B cyclo-Propylmethyl bedeutet.**

| | | | |
|---|---|---|---|
| 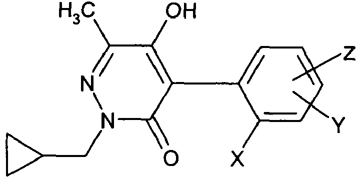 | | | |

| **Nr.** | **X** | **Y** | **Z** |
|---|---|---|---|
| 1 | F | H | H |
| 2 | Cl | H | H |
| 3 | Br | H | H |
| 4 | I | H | H |
| 5 | OMe | H | H |
| 6 | EtO | H | H |
| 7 | CF₃ | H | H |
| 8 | CN | H | H |
| 9 | NO₂ | H | H |
| 10 | OCF₃ | H | H |
| 11 | H | 3-CF₃ | H |
| 12 | H | 3-Me | H |
| 13 | H | 3-F | H |
| 14 | H | 3-Cl | H |
| 15 | H | 3-CN | H |
| 16 | H | 3-Br | H |
| 17 | H | 3-I | H |
| 18 | H | 3-NO₂ | H |
| 19 | H | 3-OCF₃ | H |
| 20 | H | 3-OMe | H |
| 21 | H | 3-EtO | H |
| 22 | H | 4-CF₃ | H |
| 23 | H | 4-Me | H |
| 24 | H | 4-F | H |
| 25 | H | 4-Cl | H |
| 26 | H | 4-CN | H |
| 27 | H | 4-Br | H |
| 28 | H | 4-I | H |
| 29 | H | 4-NO₂ | H |
| 30 | H | 4-OCF₃ | H |
| 31 | H | 4-OMe | H |
| 32 | H | 4-EtO | H |
| 33 | Cl | 4-Cl | H |
| 34 | H | 3-Cl | 4-Cl |
| 35 | Br | 4-Cl | H |
| 36 | Cl | H | 6-Cl |
| 37 | Cl | H | 6-F |
| 38 | F | H | 6-F |
| 39 | Me | 4-Cl | H |
| 40 | Me | 4-Br | H |
| 41 | Me | 4-I | H |
| 42 | Cl | 4-Cl | 6-Cl |
| 43 | Cl | 6-Me | 4-Br |
| 44 | Cl | 6-Me | 4-Cl |
| 45 | Br | 6-Me | 4-Cl |
| 46 | Br | 6-Me | 4-Br |
| 47 | OMe | 6-Me | 4-Cl |
| 48 | EtO | 6-Me | 4-Cl |
| 49 | Cl | 6-Me | 4-Br |
| 50 | Cl | 6-Et | 4-Cl |
| 51 | Br | 6-Et | 4-Cl |
| 52 | Br | 6-Et | 4-Br |
| 53 | OMe | 6-Et | 4-Cl |
| 54 | EtO | 6-Et | 4-Cl |
| 55 | Br | 4-Me | 6-Br |
| 56 | Cl | 4-Me | 6-Cl |
| 57 | OMe | 4-Me | 6-Me |
| 58 | EtO | 4-Me | 6-Me |
| 59 | OMe | 6-Et | 4-Me |
| 60 | EtO | 6-Et | 4-Me |
| 61 | Cl | 4-Me | 6-Et |
| 62 | Et | 6-Et | 4-Cl |
| 63 | Et | 6-Me | 4-Br |
| 64 | Et | 6-Et | 4-Br |
| 65 | Et | 6-Me | 4-Cl |
| 66 | Et | 6-Me | 4-Br |
| 67 | OMe | 4-Me | 6-Cl |
| 68 | EtO | 4-Me | 6-Cl |
| 69 | I | H | 4-Me |
| 70 | I | 6-Me | H |
| 71 | I | 6-Et | H |
| 72 | I | 4-Me | 6-Me |
| 73 | I | 6-Et | 4-Me |
| 74 | I | 6-Me | 4-Cl |
| 75 | I | 6-Et | 6-Cl |
| 76 | I | 6-Cl | 4-Me |
| 77 | Me | 4-I | H |
| 78 | Et | 4-I | H |
| 79 | Et | 4-I | 6-Me |
| 80 | Et | 4-I | 6-Et |
| 81 | Cl | 6-Me | 4-I |
| 82 | Cl | 6-Et | 4-I |
| 83 | c-Pr | H | H |
| 84 | c-Pr | 4-Me | H |
| 85 | c-Pr | H | 6-Me |
| 86 | c-Pr | 6-Et | H |
| 87 | c-Pr | 4-Me | 6-Me |
| 88 | c-Pr | 6-Et | 4-Me |
| 89 | c-Pr | 4-Me | 6-Cl |
| 90 | c-Pr | 6-Et | 4-Cl |
| 91 | c-Pr | 4-Cl | 6-Me |
| 92 | Me | 4-c-Pr | H |
| 93 | Et | 4-c-Pr | H |
| 94 | Me | 4-c-Pr | 6-Me |
| 95 | Et | 4-c-Pr | 6-Me |
| 96 | Et | 4-c-Pr | 6-Et |
| 97 | Cl | 6-Me | 4-c-Pr |
| 98 | Cl | 6-Et | 4-c-Pr |
| 99 | Et | 6-Et | 4-I |
| 100 | Cl | 6-F | 3-Me |
| 101 | F | 6-F | 3-F |
| 102 | EtO | 6-F | 3-F |
| 103 | F | 6-F | 3-EtO |
| 104 | F | H | 5-Cl |
| 105 | H | 3-CF₃ | 5-CF₃ |
| 106 | Me | 4-OCF₃ | H |
| 107 | OCF₃ | 4-Me | H |
| 108 | OCF₃ | 5-Me | H |
| 109 | OCF₃ | 6-Me | H |
| 110 | OCF₃ | 6-Et | H |
| 111 | Me | 5-OCF₃ | H |
| 112 | Me | 3-OCF₃ | 6-Me |
| 113 | Br | 4-OCF₃ | 6-Cl |
| 114 | Br | 4-OCF₃ | 6-Br |
| 115 | OMe | 4-OCF₃ | 6-Br |
| 116 | OMe | 4-OCF₃ | 6-Cl |
| 117 | Cl | 4-OCF₃ | 6-Cl |
| 118 | OMe | 4-OCF₃ | 6-Cl |
| 119 | OMe | 4-OCF₃ | 6-Br |
| 120 | Me | 4-OCF₃ | 6-Me |
| 121 | Cl | 4-OCF₃ | 6-Me |
| 122 | OCF₃ | 6-Cl | 4-Br |
| 123 | OCF₃ | 6-Me | 4-Me |
| 124 | OCF₃ | 6-OMe | 4-Cl |
| 125 | OCF₃ | 6-Cl | 4-Me |
| 126 | Cl | 5-OCF₃ | H |
| 127 | Br | 5-OCF₃ | H |
| 128 | OCF₃ | 6-Et | 4-Cl |
| 129 | Br | 4-Cl | 6-Br |
| 130 | Br | 4-Cl | 6-Cl |
| 131 | Br | 4-Me | 6-Cl |
| 132 | Cl | 3-Me | 6-Cl |
| 133 | Cl | 3-F | 6-F |
| 134 | F | 3-Me | 6-F |
| 135 | F | 4-OMe | 6-F |
| 136 | F | 3-OMe | 6-F |
| 137 | Cl | 3-Cl | 6-F |
| 138 | Cl | 4-Et | 6-Cl |
| 139 | Cl | 4-Et | 6-Br |
| 140 | Cl | 3-Br | 6-Cl |
| 141 | Cl | 4-CF₃ | 6-F |
| 142 | Cl | 4-CF₃ | 6-Cl |
| 143 | Cl | 3-Cl | 6-Cl |
| 144 | Cl | 3-CF₃ | 6-Cl |
| 145 | F | 3-F | 6-NO₂ |
| 146 | F | 4-NO₂ | 6-F |
| 147 | Cl | 4-CF₃ | 6-NO₂ |
| 148 | Br | 6-NO₂ | H |
| 149 | F | 4-CF₃ | 6-F |
| 150 | Br | 6-Br | H |
| 151 | Cl | 3-OMe | 6-F |
| 152 | F | 3-OMe | 6-Cl |
| 153 | F | 4-Cl | 6-F |
| 154 | F | 4-Br | 6-F |
| 155 | F | 4-Br | 6-Br |
| 156 | Cl | 4-Br | 6-Cl |
| 157 | F | 4-EtO | 6-F |
| 158 | F | 3-Cl | 6-F |
| 159 | Cl | 3-Cl | 6-Br |
| 160 | F | 3-F | 6-Cl |
| 161 | F | 3-F | 6-Br |
| 162 | F | 3-F | 6-I |
| 163 | Cl | 6-CF₃ | H |
| 164 | Cl | 3-Cl | 6-CF₃ |
| 165 | F | 3-Cl | 6-CF₃ |
| 166 | Cl | 3-CF₃ | 6-Cl |
| 167 | c-Pr | 4-Cl | 6-Cl |

Tabelle 2: Erfindungsgemäße Verbindungen der allgemeinen Formel (I), worin G für Wasserstoff steht, A für Ethyl steht, und B cyclo-Propylmethyl bedeutet und X, Y und Z, die in Tabelle 1 angegebenen Bedeutungen haben

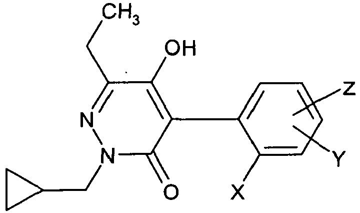

Tabelle 3: Erfindungsgemäße Verbindungen der allgemeinen Formel (I), worin G für Wasserstoff steht, A für Propyl steht, und B cyclo-Propylmethyl bedeutet und X, Y und Z, die in Tabelle 1 angegebenen Bedeutungen haben

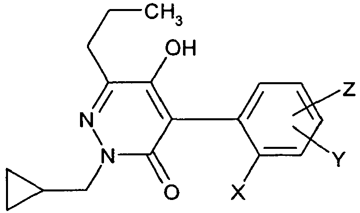

Tabelle 4: Erfindungsgemäße Verbindungen der allgemeinen Formel (I), worin G für Wasserstoff steht, A für iso-Butyl steht, und B cyclo-Propylmethyl bedeutet und X, Y und Z, die in Tabelle 1 angegebenen Bedeutungen haben

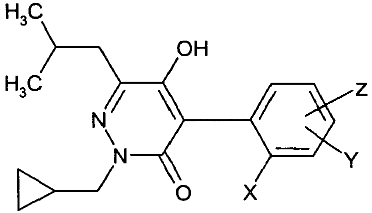

Tabelle 5: Erfindungsgemäße Verbindungen der allgemeinen Formel (I), worin G für Wasserstoff steht, A für Isopropyl steht, und B cyclo-Propylmethyl bedeutet und X, Y und Z, die in Tabelle 1 angegebenen Bedeutungen haben

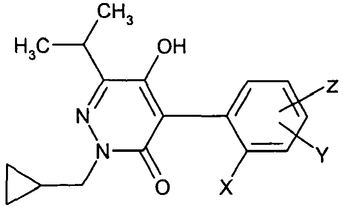

Tabelle 6: Erfindungsgemäße Verbindungen der allgemeinen Formel (I), worin G für Wasserstoff steht, A für Methyl steht, und B cyclo-Butyl bedeutet und X, Y und Z, die in Tabelle 1 angegebenen Bedeutungen haben

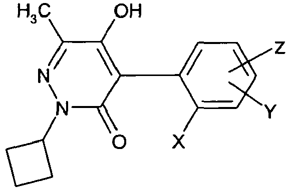

Tabelle 7: Erfindungsgemäße Verbindungen der allgemeinen Formel (I), worin G für Wasserstoff steht, A für Ethyl steht, und B cyclo-Butyl bedeutet und X, Y und Z, die in Tabelle 1 angegebenen Bedeutungen haben

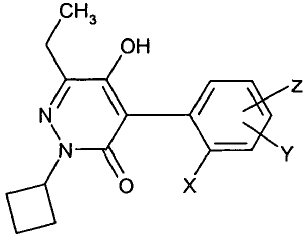

Tabelle 8: Erfindungsgemäße Verbindungen der allgemeinen Formel (I), worin G für Wasserstoff steht, A für Propyl steht, und B cyclo-Butyl bedeutet und X, Y und Z, die in Tabelle 1 angegebenen Bedeutungen haben

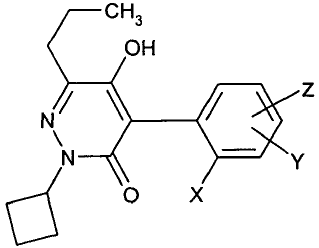

Tabelle 9: Erfindungsgemäße Verbindungen der allgemeinen Formel (I), worin G für Wasserstoff steht, und A für iso-Butyl, und B cyclo-Butyl bedeutet und X, Y und Z, die in Tabelle 1 angegebenen Bedeutungen haben

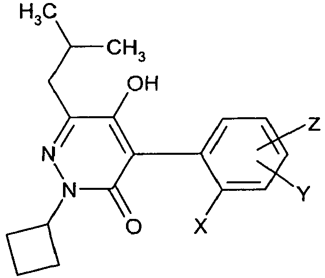

Tabelle 10: Erfindungsgemäße Verbindungen der allgemeinen Formel (I), worin G für Wasserstoff steht, und für iso-Propyl, und B cyclo-Butyl und X, Y und Z, die in Tabelle 1 angegebenen Bedeutungen haben

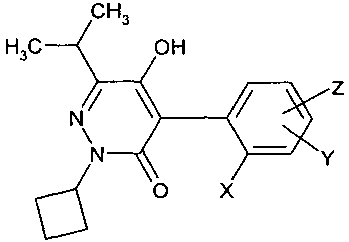

Tabelle 11: Erfindungsgemäße Verbindungen der allgemeinen Formel (I), worin G für Wasserstoff steht, A für Methyl steht, und B 2,2-Difluroethyl bedeutet und X, Y und Z, die in Tabelle 1 angegebenen Bedeutungen haben

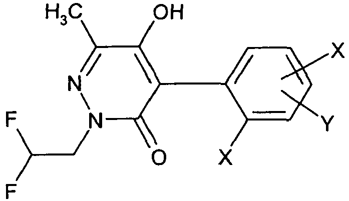

Tabelle 12: Erfindungsgemäße Verbindungen der allgemeinen Formel (I), worin G für Wasserstoff steht, A für Ethyl steht, und B 2,2-Difluroethyl bedeutet und X, Y und Z, die in Tabelle 1 angegebenen Bedeutungen haben

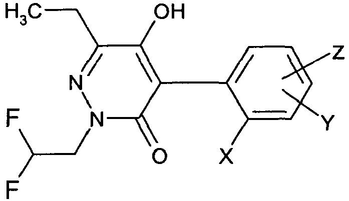

Tabelle 13: Erfindungsgemäße Verbindungen der allgemeinen Formel (I), worin G für Wasserstoff steht, A für Propyl steht, und B B 2,2-Difluroethyl bedeutet und X, Y und Z, die in Tabelle 1 angegebenen Bedeutungen haben

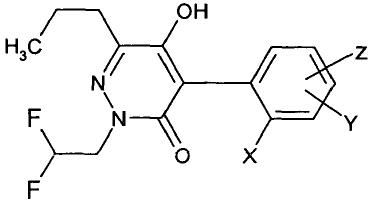

Tabelle 14: Erfindungsgemäße Verbindungen der allgemeinen Formel (I), worin G für Wasserstoff steht, A für iso-Butyl steht, und B 2,2-Difluroethyl bedeutet und X, Y und Z, die in Tabelle 1 angegebenen Bedeutungen haben

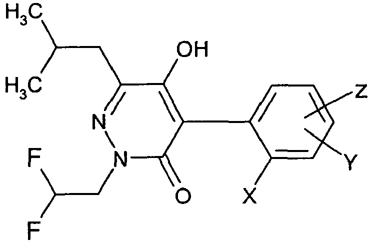

Tabelle 15: Erfindungsgemäße Verbindungen der allgemeinen Formel (I), worin G für Wasserstoff steht, A für iso-Propyl steht, und B 2,2-Difluroethyl bedeutet und X, Y und Z, die in Tabelle 1 angegebenen Bedeutungen haben

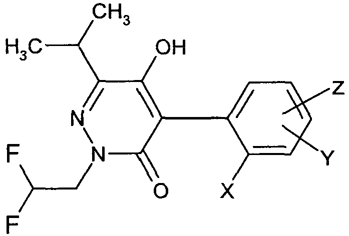

Tabelle 16: Erfindungsgemäße Verbindungen der allgemeinen Formel (I), worin G für Wasserstoff steht, A für Methyl steht, und B 2,2,2-Trifluroethyl bedeutet und X, Y und Z, die in Tabelle 1 angegebenen Bedeutungen haben

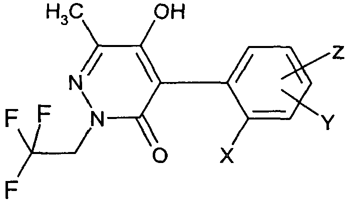

Tabelle 17: Erfindungsgemäße Verbindungen der allgemeinen Formel (I), worin G für Wasserstoff steht, A für Ethyl steht, und B 2,2,2-Trifluroethyl bedeuten und X, Y und Z, die in Tabelle 1 angegebenen Bedeutungen haben

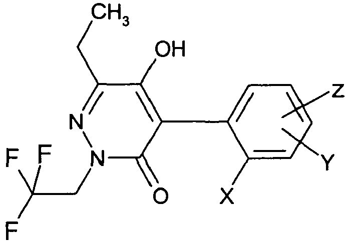

Tabelle 18: Erfindungsgemäße Verbindungen der allgemeinen Formel (I), worin G für Wasserstoff steht, A für Propyl steht, und B 2,2,2-Trifluroethyl und X, Y und Z, die in Tabelle 1 angegebenen Bedeutungen haben

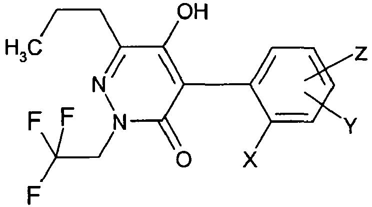

Tabelle 19: Erfindungsgemäße Verbindungen der allgemeinen Formel (I), worin G für Wasserstoff steht, und A für iso-Butyl steht, und B 2,2,2-Trifluroethyl und X, Y und Z, die in Tabelle 1 angegebenen Bedeutungen haben

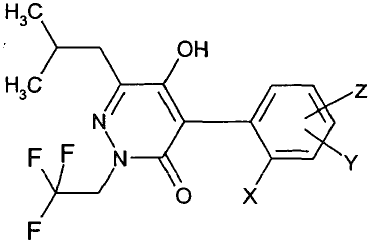

Tabelle 20: Erfindungsgemäße Verbindungen der allgemeinen Formel (I), worin G für Wasserstoff steht, A für iso-Propyl steht, und B 2,2,2-Trifluroethyl und X, Y und Z, die in Tabelle 1 angegebenen Bedeutungen haben

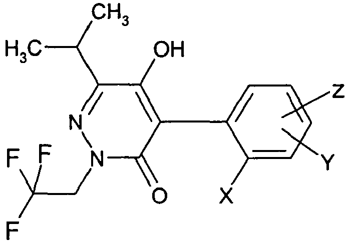

Tabelle 21: Erfindungsgemäße Verbindungen der allgemeinen Formel (I), worin G für Wasserstoff steht, A für Methyl steht, und B Benzyl bedeutet und X, Y und Z, die in Tabelle 1 angegebenen Bedeutungen haben

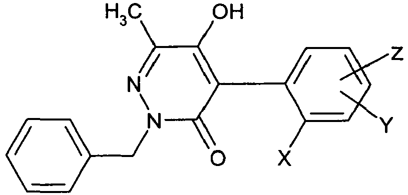

Tabelle 22: Erfindungsgemäße Verbindungen der allgemeinen Formel (I), worin G für Wasserstoff steht, A für Ethyl steht, und B Benzyl bedeutet und X, Y und Z, die in Tabelle 1 angegebenen Bedeutungen haben

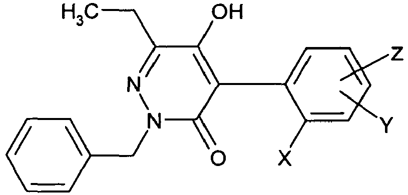

Tabelle 23: Erfindungsgemäße Verbindungen der allgemeinen Formel (I), worin G für Wasserstoff steht, A für Propyl steht, und B Benzyl bedeutet und X, Y und Z, die in Tabelle 1 angegebenen Bedeutungen haben

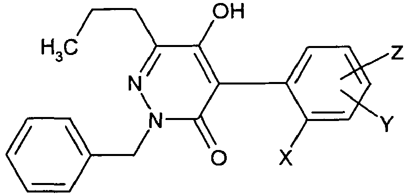

Tabelle 24: Erfindungsgemäße Verbindungen der allgemeinen Formel (I), worin G für Wasserstoff steht, A für iso-Butyl steht, und B Benzyl bedeutet. und X, Y und Z, die in Tabelle 1 angegebenen Bedeutungen haben

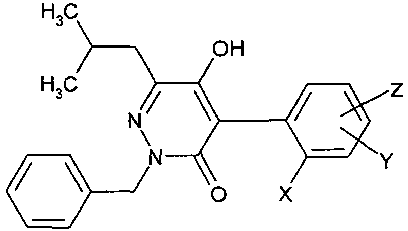

Tabelle 25: Erfindungsgemäße Verbindungen der allgemeinen Formel (I), worin G für Wasserstoff steht, A für iso-Propyl, und B Benzyl bedeutet und X, Y und Z, die in Tabelle 1 angegebenen Bedeutungen haben

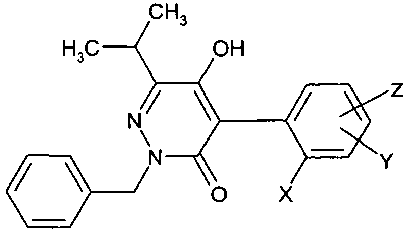

Tabelle 26: Erfindungsgemäße Verbindungen der allgemeinen Formel (I), worin G für Wasserstoff steht, A für Methyl steht, und B 2-Chlorphenylmethyl bedeutet und X, Y und Z, die in Tabelle 1 angegebenen Bedeutungen haben

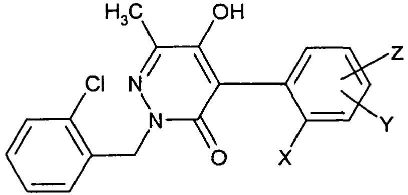

Tabelle 27: Erfindungsgemäße Verbindungen der allgemeinen Formel (I), worin G für Wasserstoff steht, A für Ethyl steht, und B 2-Chlorphenylmethyl und X, Y und Z, die in Tabelle 1 angegebenen Bedeutungen haben

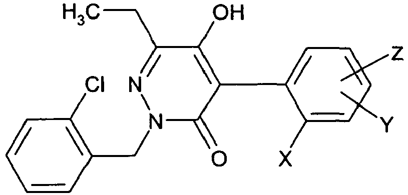

Tabelle 28: Erfindungsgemäße Verbindungen der allgemeinen Formel (I), worin G für Wasserstoff steht, A für Propyl steht, und B 2-Chlorphenylmethyl bedeutet und X, Y und Z, die in Tabelle 1 angegebenen Bedeutungen haben

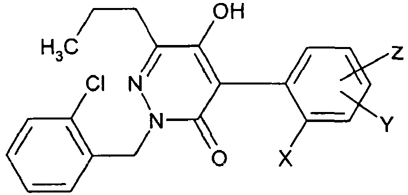

Tabelle 29: Erfindungsgemäße Verbindungen der allgemeinen Formel (I), worin G für Wasserstoff steht, A für iso-Butyl steht, und B 2-Chlorphenylmethyl bedeutet und X, Y und Z, die in Tabelle 1 angegebenen Bedeutungen haben

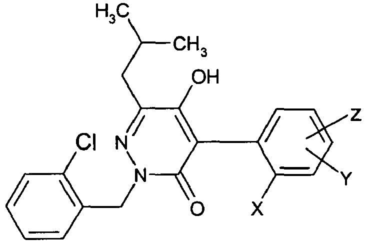

Tabelle 30: Erfindungsgemäße Verbindungen der allgemeinen Formel (I), worin G für Wasserstoff steht, A für iso-Propyl steht, und B 2-Chlorphenylmethyl bedeutet und X, Y und Z, die in Tabelle 1 angegebenen Bedeutungen haben

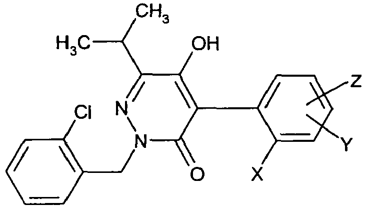

Tabelle 31: Erfindungsgemäße Verbindungen der allgemeinen Formel (I), worin G für Wasserstoff steht, A für Methyl steht, und B 3-Chlorphenylmethyl bedeutet und X, Y und Z, die in Tabelle 1 angegebenen Bedeutungen haben

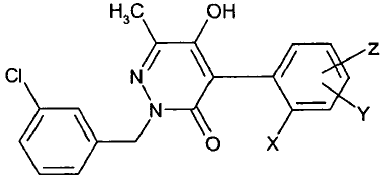

Tabelle 32: Erfindungsgemäße Verbindungen der allgemeinen Formel (I), worin G für Wasserstoff steht, A für Ethyl steht, und B 3-Chlorphenylmethyl bedeutet und X, Y und Z, die in Tabelle 1 angegebenen Bedeutungen haben

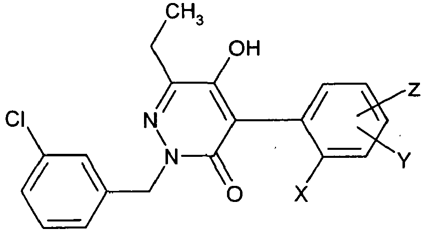

Tabelle 33: Erfindungsgemäße Verbindungen der allgemeinen Formel (I), worin G für Wasserstoff steht, A für Propyl steht, und B 3-Chlorphenylmethyl bedeutet und X, Y und Z, die in Tabelle 1 angegebenen Bedeutungen haben

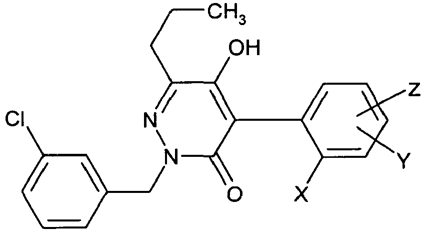

Tabelle 34: Erfindungsgemäße Verbindungen der allgemeinen Formel (I), worin G für Wasserstoff steht, A für iso-Butyl steht, und B 3-Chlorphenylmethyl bedeutet und X, Y und Z, die in Tabelle 1 angegebenen Bedeutungen haben

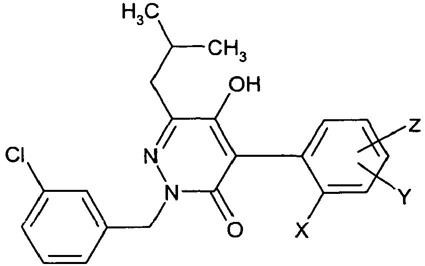

Tabelle 35: Erfindungsgemäße Verbindungen der allgemeinen Formel (I), worin G für Wasserstoff steht, A für iso-Propyl steht, und B 3-Chlorphenylmethyl bedeutet und X, Y und Z, die in Tabelle 1 angegebenen Bedeutungen haben

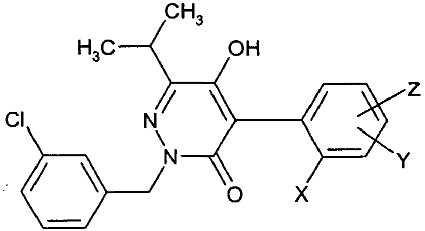

Tabelle 36: Erfindungsgemäße Verbindungen der allgemeinen Formel (I), worin G für Wasserstoff steht, A für Methyl steht, und B 4-Chlorphenylmethyl bedeutet und X, Y und Z, die in Tabelle 1 angegebenen Bedeutungen haben

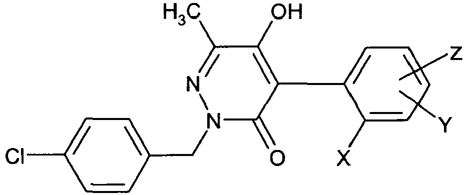

Tabelle 37: Erfindungsgemäße Verbindungen der allgemeinen Formel (I), worin G für Wasserstoff steht, A für Ethyl steht, und B 4-Chlorphenylmethyl bedeutet und X, Y und Z, die in Tabelle 1 angegebenen Bedeutungen haben

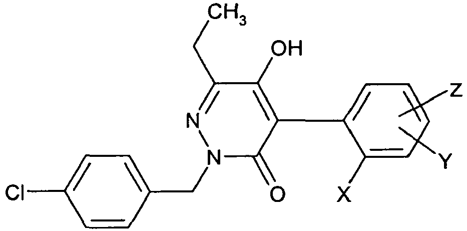

Tabelle 38: Erfindungsgemäße Verbindungen der allgemeinen Formel (I), worin G für Wasserstoff steht, A für Propyl steht, und B 4-Chlorphenylmethyl bedeutet und X, Y und Z, die in Tabelle 1 angegebenen Bedeutungen haben

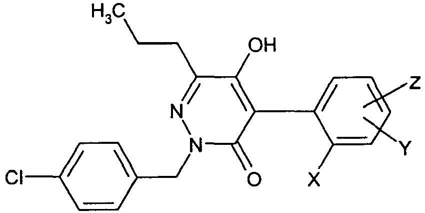

Tabelle 39: Erfindungsgemäße Verbindungen der allgemeinen Formel (I), worin G für Wasserstoff steht, A für iso-Butyl steht, und B 4-Chlorphenylmethyl bedeutet und X, Y und Z, die in Tabelle 1 angegebenen Bedeutungen haben

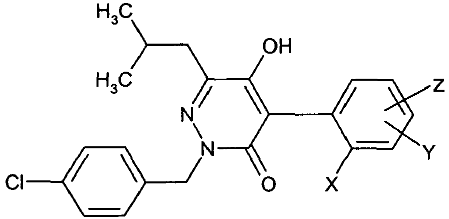

Tabelle 40: Erfindungsgemäße Verbindungen der allgemeinen Formel (I), worin G für Wasserstoff steht, A für iso-Propyl steht, und B 4-Chlorphenylmethyl bedeutet und X, Y und Z, die in Tabelle 1 angegebenen Bedeutungen haben

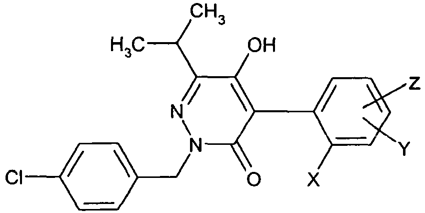

Tabelle 41: Erfindungsgemäße Verbindungen der allgemeinen Formel (I), worin G für Wasserstoff steht, A für Methyl steht, und B 2-Phenylethyl bedeutet und X, Y und Z, die in Tabelle 1 angegebenen Bedeutungen haben

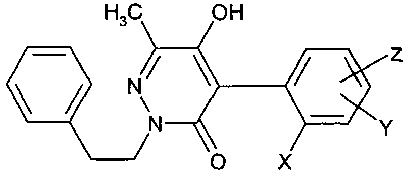

Tabelle 42: Erfindungsgemäße Verbindungen der allgemeinen Formel (I), worin G für Wasserstoff steht, A für Ethyl steht, und B 2-Phenylethyl bedeutet und X, Y und Z, die in Tabelle 1 angegebenen Bedeutungen haben

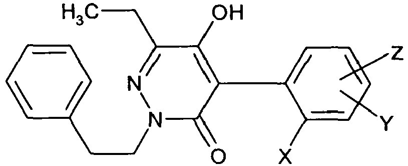

Tabelle 43: Erfindungsgemäße Verbindungen der allgemeinen Formel (I), worin G für Wasserstoff steht, A für Propyl steht, und B 2-Phenylethyl bedeutet und X, Y und Z, die in Tabelle 1 angegebenen Bedeutungen haben

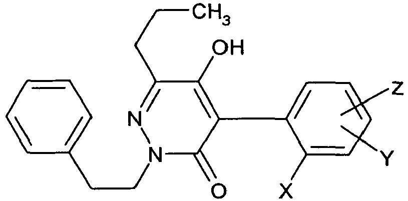

Tabelle 44: Erfindungsgemäße Verbindungen der allgemeinen Formel (I), worin G für Wasserstoff steht, A für iso-Butyl steht, und B 2-Phenylethyl bedeutet und X, Y und Z, die in Tabelle 1 angegebenen Bedeutungen haben

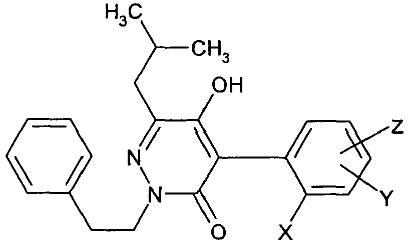

Tabelle 45: Erfindungsgemäße Verbindungen der allgemeinen Formel (I), worin G für Wasserstoff steht, A für iso-Propyl steht, und B 2-Phenylethyl bedeutet und X, Y und Z, die in Tabelle 1 angegebenen Bedeutungen haben.

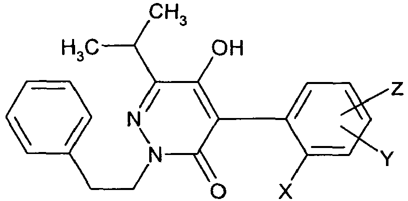

Tabelle 46: Erfindungsgemäße Verbindungen der allgemeinen Formel (I), worin G für Wasserstoff steht, A für Methyl steht, und B 2-(2-Chlorphenyl)-ethyl bedeutet und X, Y und Z, die in Tabelle 1 angegebenen Bedeutungen haben.

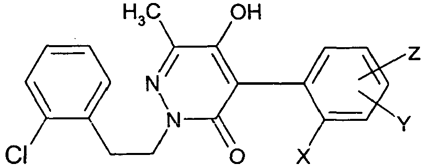

Tabelle 47: Erfindungsgemäße Verbindungen der allgemeinen Formel (I), worin G für Wasserstoff steht, A für Ethyl steht, und B 2-(2-Chlorphenyl)-ethyl bedeutet und X, Y und Z, die in Tabelle 1 angegebenen Bedeutungen haben.

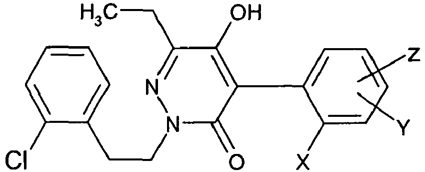

Tabelle 48: Erfindungsgemäße Verbindungen der allgemeinen Formel (I), worin G für Wasserstoff steht, A für Ethyl steht, und B 2-(2-Chlorphenyl)-ethyl bedeutet und X, Y und Z, die in Tabelle 1 angegebenen Bedeutungen haben.

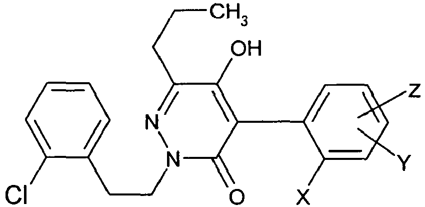

Tabelle 49: Erfindungsgemäße Verbindungen der allgemeinen Formel (I), worin G für Wasserstoff steht, A für iso-Butyl steht, und B 2-(2-Chlorphenyl)-ethyl bedeutet und X, Y und Z, die in Tabelle 1 angegebenen Bedeutungen haben.

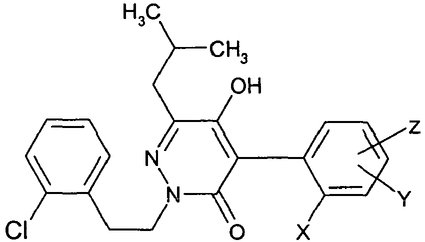

Tabelle 50: Erfindungsgemäße Verbindungen der allgemeinen Formel (I), worin G für Wasserstoff steht, A für iso-Propyl steht, und B 2-(2-Chlorphenyl)-ethyl bedeutet und X, Y und Z, die in Tabelle 1 angegebenen Bedeutungen haben.

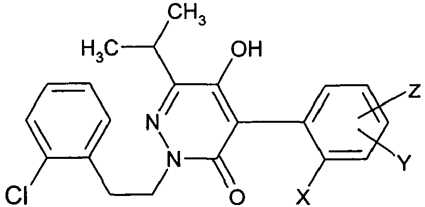

Tabelle 51: Erfindungsgemäße Verbindungen der allgemeinen Formel (I), worin G für Wasserstoff steht, A für Methyl steht, und B 2-(4-Chlorphenyl)-ethyl bedeutet und X, Y und Z, die in Tabelle 1 angegebenen Bedeutungen haben.

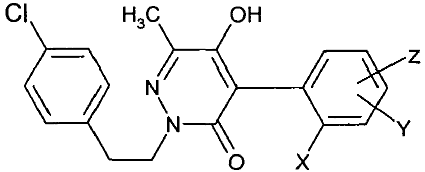

Tabelle 52: Erfindungsgemäße Verbindungen der allgemeinen Formel (I), worin G für Wasserstoff steht, A für Ethyl steht, und B 2-(4-Chlorphenyl)-ethyl bedeutet und X, Y und Z, die in Tabelle 1 angegebenen Bedeutungen haben.

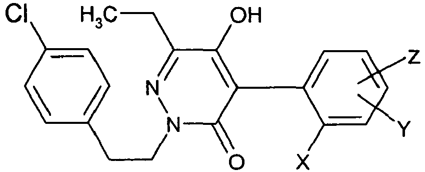

Tabelle 53: Erfindungsgemäße Verbindungen der allgemeinen Formel (I), worin G für Wasserstoff steht, A für Propyl steht, und B 2-(4-Chlorphenyl)-ethyl bedeutet und X, Y und Z, die in Tabelle 1 angegebenen Bedeutungen haben.

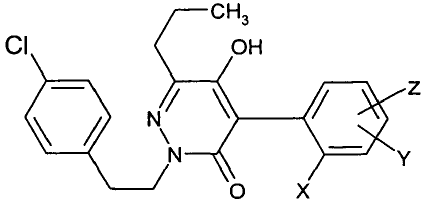

Tabelle 54: Erfindungsgemäße Verbindungen der allgemeinen Formel (I), worin G für Wasserstoff steht, A für iso-Butyl steht, und B 2-(4-Chlorphenyl)-ethyl bedeutet und X, Y und Z, die in Tabelle 1 angegebenen Bedeutungen haben.

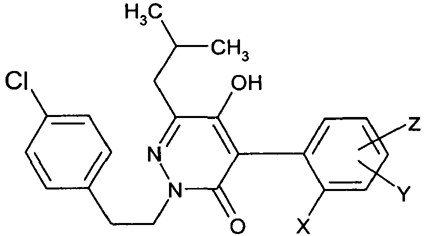

Tabelle 55: Erfindungsgemäße Verbindungen der allgemeinen Formel (I), worin G für Wasserstoff steht, A für iso-Propyl steht, und B 2-(4-Chlorphenyl)-ethyl bedeutet und X, Y und Z, die in Tabelle 1 angegebenen Bedeutungen haben.

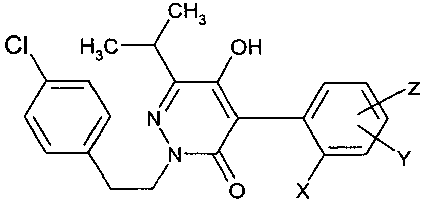

Tabelle 56: Erfindungsgemäße Verbindungen der allgemeinen Formel (I), worin G für Wasserstoff steht, A für c-Propyl steht, und B 4Methyl bedeutet und X, Y und Z, die in Tabelle 1 angegebenen Bedeutungen haben.

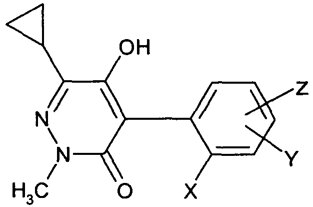

Tabelle 57: Erfindungsgemäße Verbindungen der allgemeinen Formel (I), worin G für Wasserstoff steht, A für c-Propyl steht, und B Ethyl bedeutet und X, Y und Z, die in Tabelle 1 angegebenen Bedeutungen haben.

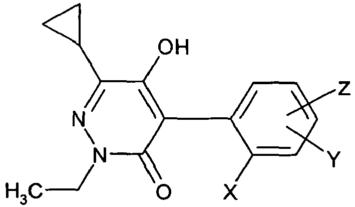

Tabelle 58: Erfindungsgemäße Verbindungen der allgemeinen Formel (I), worin G für Wasserstoff steht, A für c-Propyl steht, und iso-Butyl bedeutet und X, Y und Z, die in Tabelle 1 angegebenen Bedeutungen haben.

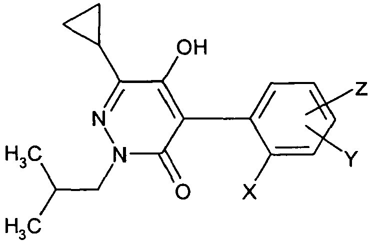

Tabelle 59: Erfindungsgemäße Verbindungen der allgemeinen Formel (I), worin G für Wasserstoff steht, A für c-Pentyl steht, und B Methyl bedeutet und X, Y und Z, die in Tabelle 1 angegebenen Bedeutungen haben.

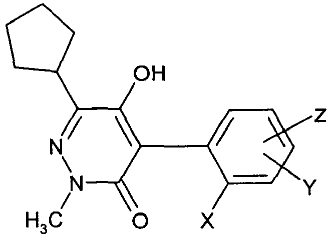

Tabelle 60: Erfindungsgemäße Verbindungen der allgemeinen Formel (I), worin G für Wasserstoff steht, A für c-Pentyl steht, und B Ethyl bedeutet und X, Y und Z, die in Tabelle 1 angegebenen Bedeutungen haben.

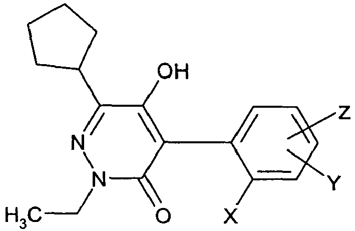

Tabelle 61: Erfindungsgemäße Verbindungen der allgemeinen Formel (I), worin G für Wasserstoff steht, A für c-Pentyl steht, und B iso-Butyl bedeutet und X, Y und Z, die in Tabelle 1 angegebenen Bedeutungen haben.

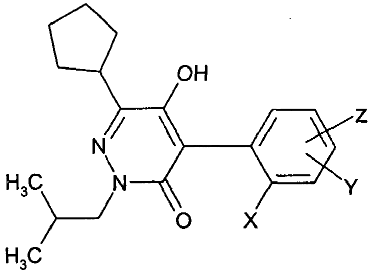

Tabelle 62: Erfindungsgemäße Verbindungen der allgemeinen Formel (I), worin G für Wasserstoff steht, A für Benzyl steht, und B Methyl bedeutet und X, Y und Z, die in Tabelle 1 angegebenen Bedeutungen haben.

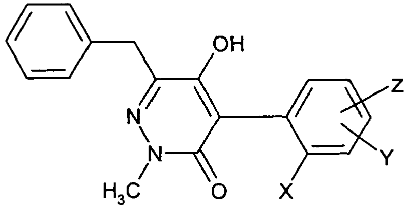

Tabelle 63: Erfindungsgemäße Verbindungen der allgemeinen Formel (I), worin G für Wasserstoff steht, A für Benzyl steht, und B Ethyl bedeutet und X, Y und Z, die in Tabelle 1 angegebenen Bedeutungen haben.

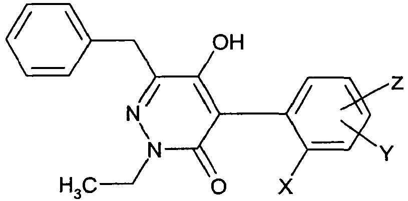

Tabelle 64: Erfindungsgemäße Verbindungen der allgemeinen Formel (I), worin G für Wasserstoff steht, A Benzyl steht, und B iso-Butyl bedeutet und X, Y und Z, die in Tabelle 1 angegebenen Bedeutungen haben.

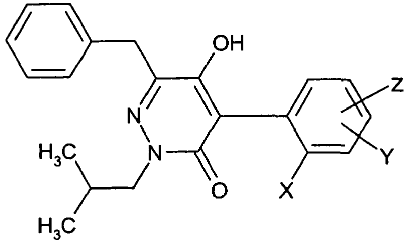

Tabelle 65: Erfindungsgemäße Verbindungen der allgemeinen Formel (I), worin G für Wasserstoff steht, A für 4-Fluorbenzyl steht, und B Methyl bedeutet und X, Y und Z, die in Tabelle 1 angegebenen Bedeutungen haben.

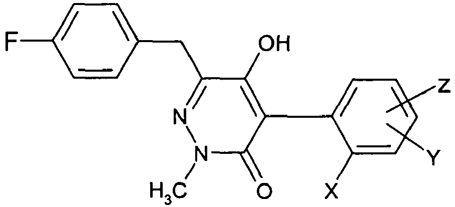

Tabelle 66: Erfindungsgemäße Verbindungen der allgemeinen Formel (I), worin G für Wasserstoff steht, A für 4-Fluorbenzyl steht, und B Ethyl bedeutet und X, Y und Z, die in Tabelle 1 angegebenen Bedeutungen haben.

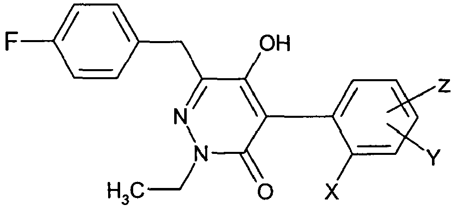

Tabelle 67: Erfindungsgemäße Verbindungen der allgemeinen Formel (I), worin G für Wasserstoff steht, A für 4-Fluorbenzyl steht, und B iso-Butyl bedeutet und X, Y und Z, die in Tabelle 1 angegebenen Bedeutungen haben.

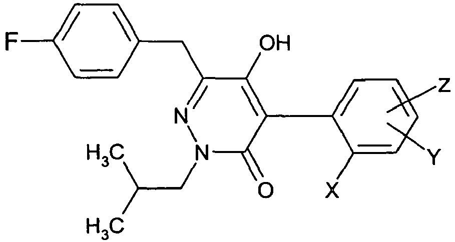

Tabelle 68: Erfindungsgemäße Verbindungen der allgemeinen Formel (I), worin G für Wasserstoff steht, A für 2-Fluorbenzyl steht, und B Methyl bedeutet und X, Y und Z, die in Tabelle 1 angegebenen Bedeutungen haben.

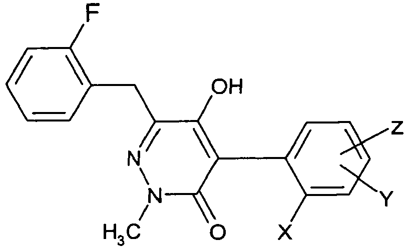

Tabelle 69: Erfindungsgemäße Verbindungen der allgemeinen Formel (I), worin G für Wasserstoff steht, A für 2-Fluorbenzyl steht, und B Ethyl bedeutet und X, Y und Z, die in Tabelle 1 angegebenen Bedeutungen haben.

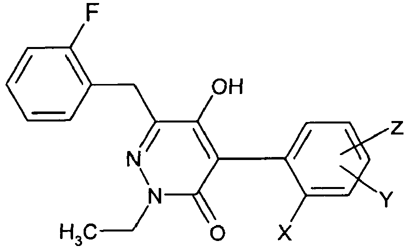

Tabelle 70: Erfindungsgemäße Verbindungen der allgemeinen Formel (I), worin G für Wasserstoff steht, A für 2-Fluorbenzyl steht, und B iso-Butyl bedeutet und X, Y und Z, die in Tabelle 1 angegebenen Bedeutungen haben.

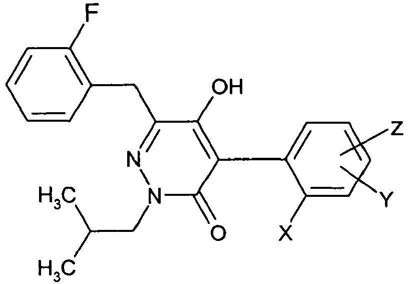

Tabelle 71: Erfindungsgemäße Verbindungen der allgemeinen Formel (I), worin G für Wasserstoff steht, A für 3-Fluorbenzyl steht, und B Methyl bedeutet und X, Y und Z, die in Tabelle 1 angegebenen Bedeutungen haben.

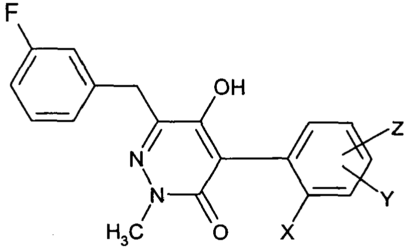

Tabelle 72: Erfindungsgemäße Verbindungen der allgemeinen Formel (I), worin G für Wasserstoff steht, A für 3-Fluorbenzyl steht, und B Ethyl bedeutet und X, Y und Z, die in Tabelle 1 angegebenen Bedeutungen haben.

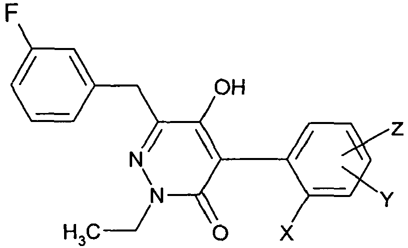

Tabelle 73: Erfindungsgemäße Verbindungen der allgemeinen Formel (I), worin G für Wasserstoff steht, A für 3-Fluorbenzyl steht, und B iso-Butyl bedeutet und X, Y und Z, die in Tabelle 1 angegebenen Bedeutungen haben.

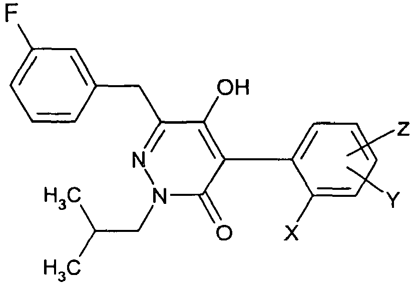

Tabelle 74: Erfindungsgemäße Verbindungen der allgemeinen Formel (I), worin G für Wasserstoff steht, A für 2-Phenylethyl steht, und B 4-Methyl bedeutet und X, Y und Z, die in Tabelle 1 angegebenen Bedeutungen haben.

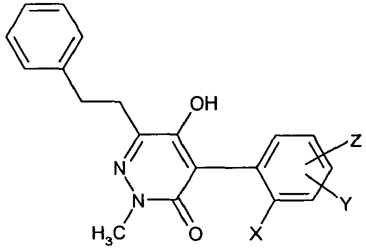

Tabelle 75: Erfindungsgemäße Verbindungen der allgemeinen Formel (I), worin G für Wasserstoff steht, A für 2-Phenylethyl steht, und B 4-Ethyl bedeutet und X, Y und Z, die in Tabelle 1 angegebenen Bedeutungen haben.

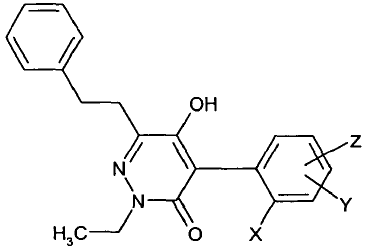

Tabelle 76: Erfindungsgemäße Verbindungen der allgemeinen Formel (I), worin G für Wasserstoff steht, A für 2-Phenylethyl steht, und B 4-iso-Butyl bedeutet und X, Y und Z, die in Tabelle 1 angegebenen Bedeutungen haben.

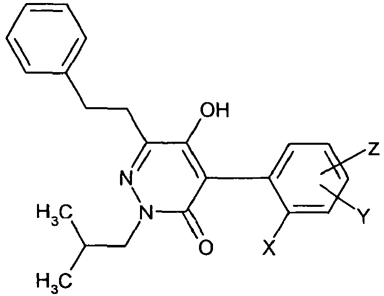

Tabelle 77: Erfindungsgemäße Verbindungen der allgemeinen Formel (I), worin G für Wasserstoff steht, A für 2-(4-Chlorphenyl)-ethyl steht, und B 4-Methyl bedeutet und X, Y und Z, die in Tabelle 1 angegebenen Bedeutungen haben.

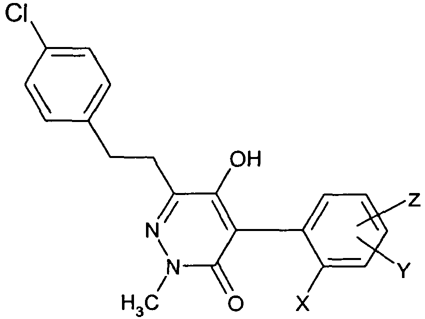

Tabelle 78: Erfindungsgemäße Verbindungen der allgemeinen Formel (I), worin G für Wasserstoff steht, A für 2-(4-Chlorphenyl)-ethyl steht, und B 4-Ethyl bedeutet und X, Y und Z, die in Tabelle 1 angegebenen Bedeutungen haben.

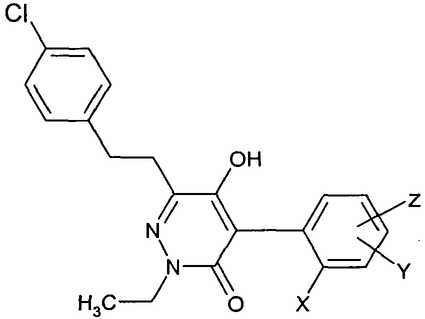

Tabelle 79: Erfindungsgemäße Verbindungen der allgemeinen Formel (I), worin G für Wasserstoff steht, A für 2-(4-Chlorphenyl)-ethyl steht, und B 4-iso-Butyl bedeutet und X, Y und Z, die in Tabelle 1 angegebenen Bedeutungen haben.

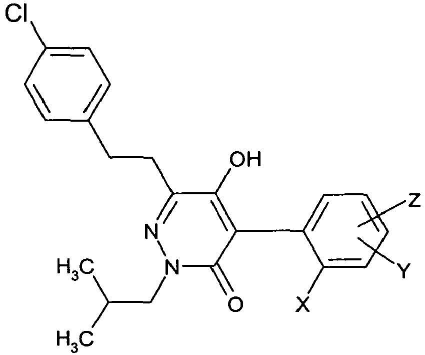

Tabelle 80: Erfindungsgemäße Verbindungen der allgemeinen Formel (I), worin G für Wasserstoff steht, A für 2-(2-Chlorphenyl)-ethyl steht, und B 4-Methyl bedeutet und X, Y und Z, die in Tabelle 1 angegebenen Bedeutungen haben.

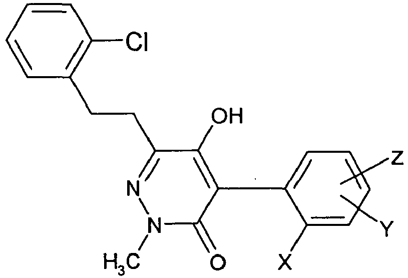

Tabelle 81: Erfindungsgemäße Verbindungen der allgemeinen Formel (I), worin G für Wasserstoff steht, A für 2-(2-Chlorphenyl)-ethyl steht, und B 4-Ethyl bedeutet und X, Y und Z, die in Tabelle 1 angegebenen Bedeutungen haben.

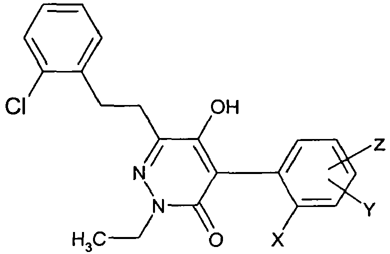

Tabelle 82: Erfindungsgemäße Verbindungen der allgemeinen Formel (I), worin G für Wasserstoff steht, A 2-(4-Chlorphenyl)-ethyl steht, und B 4-iso-Butyl bedeutet und X, Y und Z, die in Tabelle 1 angegebenen Bedeutungen haben.

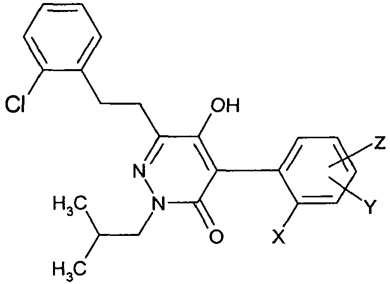

Tabelle 83: Erfindungsgemäße Verbindungen der allgemeinen Formel (I), worin G für Wasserstoff steht, A für 2-(3-Chlorphenyl)-ethyl steht, und B 4-Methyl bedeutet und X, Y und Z, die in Tabelle 1 angegebenen Bedeutungen haben.

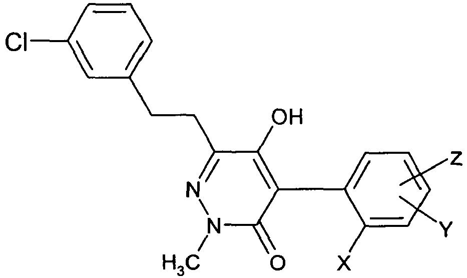

Tabelle 84: Erfindungsgemäße Verbindungen der allgemeinen Formel (I), worin G für Wasserstoff steht, A für 2-(3-Chlorphenyl)-ethyl steht, und B 4-Ethyl bedeutet und X, Y und Z, die in Tabelle 1 angegebenen Bedeutungen haben.

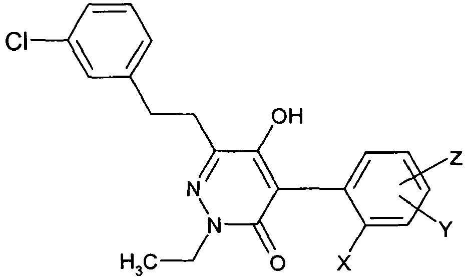

Tabelle 85: Erfindungsgemäße Verbindungen der allgemeinen Formel (I), worin G für Wasserstoff steht, A für 2-(3-Chlorphenyl)-ethyl steht, und B 4-iso-Butyl bedeutet und X, Y und Z, die in Tabelle 1 angegebenen Bedeutungen haben.

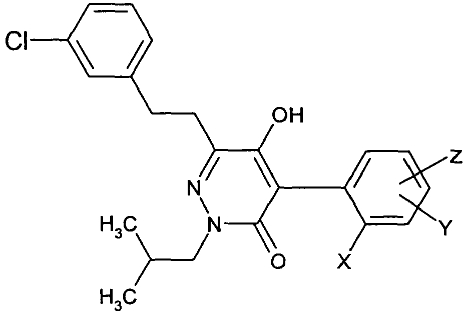

Tabelle 86: Erfindungsgemäße Verbindungen der allgemeinen Formel (I), worin G für Wasserstoff steht, A für c-Propyl steht, und B cyclo-Propylmethyl bedeutet und X, Y und Z, die in Tabelle 1 angegebenen Bedeutungen haben.

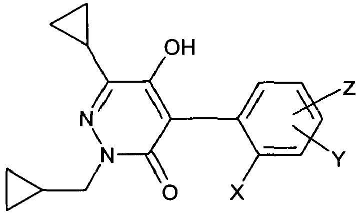

Tabelle 87: Erfindungsgemäße Verbindungen der allgemeinen Formel (I), worin G für Wasserstoff steht, A für c-Propyl steht, und B 2,2 Difluorethyl bedeutet und X, Y und Z, die in Tabelle 1 angegebenen Bedeutungen haben.

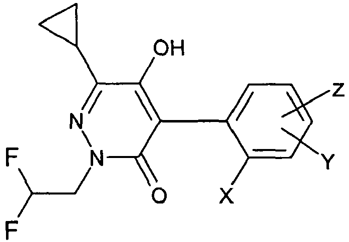

Tabelle 88: Erfindungsgemäße Verbindungen der allgemeinen Formel (I), worin G für Wasserstoff steht, A für c-Propyl steht, und B 4-2,2,2 Trifluorethyl bedeutet und X, Y und Z, die in Tabelle 1 angegebenen Bedeutungen haben.

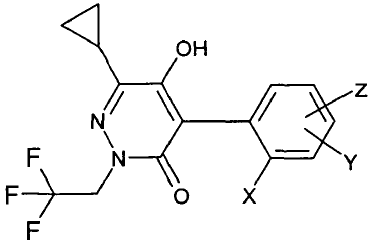

Tabelle 89: Erfindungsgemäße Verbindungen der allgemeinen Formel (I), worin G für Wasserstoff steht, A für c-Propyl steht, und B Methyl bedeutet und X, Y und Z, die in Tabelle 1 angegebenen Bedeutungen haben.

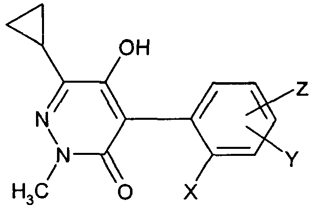

Tabelle 90: Erfindungsgemäße Verbindungen der allgemeinen Formel (I), worin G für Wasserstoff steht, A für c-Propyl steht, und B Ethyl bedeutet und X, Y und Z, die in Tabelle 1 angegebenen Bedeutungen haben.

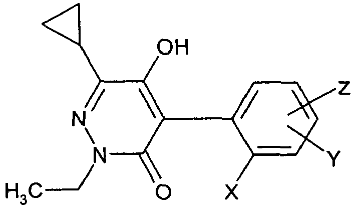

Ebenfalls ganz besonders bevorzugt sind Verbindungen der vorstehend genannten Tabellen 1 bis 32, worin G jeweils für C(=O)R¹, C(=L)LR², SO₂R³, P(=L)R⁴R⁵, C(=L)NR⁶R⁷, E oder R⁸ steht.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda,

Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 14th edition, The British Crop Protection Council and the Royal Soc. of Chemistry, 2003 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, BAS-800H, Beflubutamid, Benazolin, Benazolin-ethyl, bencarbazone, Benfluralin, Benfuresate, Bensulide, Bensulfuron-methyl, Bentazone, Benzfendizone, Benzobicyclon, Benzofenap, Benzofluor, Benzoylprop, Bifenox, Bilanafos, Bilanafosnatrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3fluorpropyl)-4,5-dihydro-5-oxo-1H-tetrazol-1-yl]-phenyl]-ethansulfonamid, Fenoprop, Fenoxaprop, Fenoxaprop-P, Fenoxaprop-ethyl, Fenoxaprop-P-ethyl, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, L-Glufosinate, L-Glufosinate-ammonium, Glufosinate-ammonium, Glyphosate, Glyphosate-isopropylammonium, H-9201, Halosafen, Halosulfuron, Halosulfuronmethyl, Haloxyfop, Haloxyfop-P, Haloxyfop-ethoxyethyl, Haloxyfop -P-ethoxyethyl, Haloxyfop-methyl, Haloxyfop-P-methyl, Hexazinone, HNPC-9908, HOK-201, HW-02, Imazamethabenz, Imazamethabenz-methyl, Imazamox, Imazapic, Imazapyr, Imazaquin, Imazethapyr, Imazosulfuron, Inabenfide, Indanofan, Indaziflam, Indolessigsäure (IAA), 4-Indol-3-ylbuttersäure (IBA), lodosulfuron, lodosulfuron-methyl-natrium, loxynil, Ipfencarbazone, Isocarbamid, Isopropalin, Isoproturon, Isouron, Isoxaben, Isoxachlortole, Isoxaflutole, Isoxapyrifop, KUH-043, KUH-071, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Methazole, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monuron, MT 128, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-dichlorobenzoyl)-1-methyl-5-benzyloxypyrazole, NC-620, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron-ethyl, Pyrazoxyfen, Pyribambenz, Pyribambenz-isopropyl, Pyribenzoxim, Pyributicarb, Pyridafol, Pyridate, Pyriftalid, Pyriminobac, Pyriminobacmethyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosatetrimesium), Sulfosulfuron, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, TH-547, d.h. Propyrisulfuron, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0166, ZJ-0270, ZJ-0543, ZJ-0862 sowie die folgenden Verbindungen

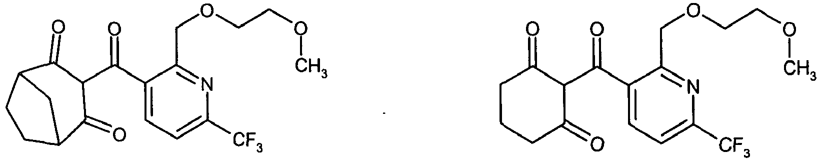

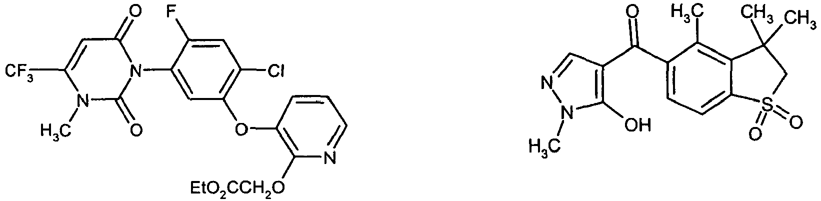

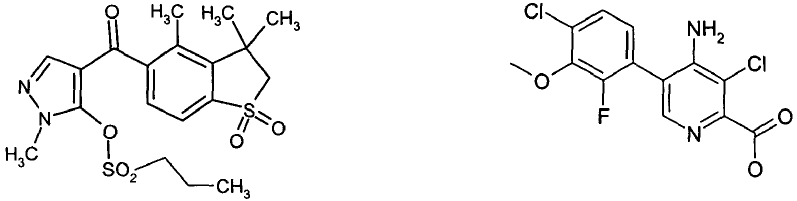

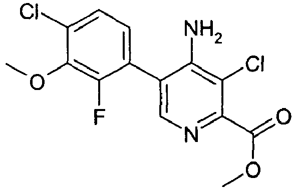

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Neben der herbiziden Wirkung weisen die erfindungsgemäßen Verbindungen auch gute insektizide Wirkung auf. Ein weiterer Gegenstand vorliegender Erfindung ist daher ihre Verwendung als Insektizide.

Die nachstehenden Beispiele erläutern die Erfindung.

### Chemische Beispiele

### 1. Herstellung von 2-Cyclobutyl-4-(2,4-dichloro-phenyl)-5-hydroxy-6-methyl-2H-pyridazin-3-on (Nr. 1 der Tabelle 91)

Zu einer Lösung von 0.331 g (2 eq) Kalium-t-butylat in 10 ml DMF wurde eine Lösung von 10ml DMF und 0,53 g (2,0 mmol) 2-Cyclobutyl-{[2-(2, 4-dichlorophenyl)-acetyl]-hydrazono}-propionsäureethyl ester bei <0° über 2 Stunden zugetropft. Man ließ 90min bei 0° rühren. und goss die Reaktionslösung auf 100ml gekühlte 0.5 N Salzsäure. Die Lösung wurde unter vermindertem Druck eingeengt und erneut in Wasser aufgenommen,wiederum eingeengt und in 50 ml Wasser aufgenommen. Man extrahierte zweimal mit je 50 ml Ethylacetat und trocknete die vereinigten organischen Phasen mit Natriumsulfat, engte unter vermindertem Druck ein und reinigte säulenchromatographisch (Silicagel, n-Heptan/EtOAc Gradient) Man erhielt so 0,1 g sauberes Produkt.

Analog den vorstehend genannten Beispiel 1 wurden folgende Verbindungen hergestellt:

**Tabelle 91: Erfindungsgemäße Verbindungen der allgemeinen Formel (I), worin G für Wasserstoff steht.**

| | | | | | | |
|---|---|---|---|---|---|---|
| 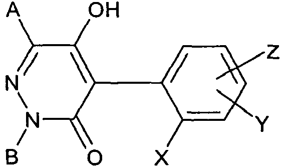 | | | | | | |

| **Nr.** | **X** | **Y** | **Z** | **A** | **B** | **Analytische Daten** |
|---|---|---|---|---|---|---|
| I-1-a-1 | Cl | 4-Cl | H | Me | c-Butyl | ¹H-NMR, 400MHz, d6-DMSO, 7.68 (d, 1H); 7.45 (dd, 1H), 7.29 (d, 1H); 5.29 (qunitett, 1H); 2.40 (m, 2H), 2,28 (s, 3H), 2.19(m ,2H), 1.75 (m, 2H) |
| I-1-a-2 | Cl | 4-Cl | 6-Et | *c*-Pentyl | Me | ¹H-NMR, 400MHz, d6-DMSO, 7.51 (d, 1H); 7.37 (d, 1H); 3.57 (s, 3H); 3.28 (m, 1H), 2.30 (m, 2H), 1.92 (m, 2H), 1.55-1.80 (m ,6H), 1.00 (t, 3H) |
| I-1-a-3 | Cl | 4-Cl | 6-Et | Me | Benzyl | |
| I-1-a-4 | Cl | 4-Me | 6-Me | Me | c-Pr-CH₂ | ¹H-NMR, 400MHz, d6-DMSO, 7.08 (d, 1H); 6.93 (d, 1H); 3.72 (d, 2H); 2.25 (s, 3H), 2.15 (s, 3H), 2.02 (d, 3H), 1.00 (m , 1 H), 0.40 (m, 2H), 0.22 (m, 2H) |
| I-1-a-5 | Cl | 6-Cl | H | c-Pentyl | Me | ¹H-NMR, 400MHz, d6-DMSO, 10.7 (bs, 1H), 7.52 (d, 2H); 7.44 (d, 1H); 3.56 (s, 3H); 3.30 (m, 1H), 2.30 (m, 2H), 1.94 (m, 2H), 1.55 - 1.80 (m ,6H) |
| I-1-a-6 | Cl | 4-Cl | 6-Et | Me | c-Pr-CH₂ | ¹H-NMR, 400MHz, d6-DMSO, 10.5 (bs, 1H), 7.51 (d, 1H); 7.35 (d, 1H); 3.88 (d, 2H); 2.33 (m, 2H), 2.25 (s, 3H), 1.22 (m, 1H), 1.03 (t ,3H), 0.45 (m, 2H), 0.32 (m, 2H) |
| I-1-a-7 | Cl | 6-Cl | H | Me | 2-Phenylethyl | ¹H-NMR, 400MHz, d6-DMSO, 10.8 (bs, 1H), 7.52 (d, 2H); 7.42 (t, 1H); 7.28-7.12 (m, 5H), 4.22 (t, 2H); 2.98 (t, 2H), 2.22 (s, 3H), |
| I-1-a-8 | Cl | 6-Cl | H | Me | 2-(2'-Chlorphenyl )-ethyl | ¹H-NMR, 400MHz, d6-DMSO, 10.8 (bs, 1H), 7.52 (d, 2H); 7.42 (m, 3H); 7.20 (m, 2H), 4.25 (t, 2H); 3.11 (t, 2H), 2.15 (s, 3H), |
| I-1-a-9 | I | H | H | Me | 2-Phenylethyl | ¹H-NMR, 400MHz, d6-DMSO, 10.4 (bs, 1H), 7.91 (d, 1H), 7.43 (t, 1H); 7.27 (m, 2H); 7.23 - 7.10-(m, 5H), 4.18 (m, 2H); 2.98 (t, 2H), 2.21 (s, 3H), |
| I-1-a-10 | I | H | H | Me | 2-(2'-Chlorphenyl )-ethyl | ¹H-NMR, 400MHz, d6-DMSO, 10.4 (bs, 1H), 7.90 (d, 1H), 7.41 (m, 2H); 7.27 (m, 3H); 7.12 (m, 2H), 4.22 (m, 2H); 3.11 (m, 2H), 2.16 (s, 3H), |
| I-1-a-11 | Cl | 4-Cl | 6-Et | c-Hexyl-CH₂ | Me | ¹H-NMR, 400MHz, d6-DMSO, 7.53 (d, 1 H); 7.37 (d, 1 H); 3.58 (s, 3H); 2.50 (m, 2H, lösungsmittelüberlagert), 2.30 (m, 2H), 1.70 (m, 6H), 1.30 - 0.90 (Signalhaufen, 9H) |
| I-1-a-12 | Cl | 4-Cl | H | c-Pentyl-CH₂ | Me | ¹H-NMR, 400MHz, d6-DMSO, 10.4 (bs, 1H), 7.70 (s, 1H), 7.41 (dd, 1 H); 7.31 (d, 1H); 3.58 (s, 3H), 2.63 (d, 2H); 2.24 (m, 1H), 1.8 - 1.2 (Signalhaufen, 8H), |
| I-1-a-13 | Cl | 6-Cl | 4-Cl | Me | Bz | ¹H-NMR, 400MHz, d6-DMSO, 7.65 (s, 2H); 7.22-7.34 (m, 5H); 5,18 (s, 2H); 2,21 (s, 3H) |
| I-1-a-14 | Cl | 6-F | 3-Me | Me | Bz | ¹-NMR, 400MHz, d6-DMSO, 7.23-7.34 (m, 7H); 5,18 (s, 2H); 2,24 (s, 6H) |
| I-1 -a-1 5 | F | 6-F | 3-F | Me | Bz | ¹H-NMR, 400MHz, d6-DMSO, 7.49 (ddd, 1H); 7.24-7.34 (m, 5H); 7.10-7.16 (m, 1H); 5,18 (s, 2H); 2,25 (s, 3H) |
| I-1-a-16 | EtO | 6-F | 3-F | Me | Bz | ¹H-NMR, 600MHz, d6-DMSO, 7.24-7.38 (m, 6H), 7.02 (ddd, 1H), 5.28 (d, 1H), 5.11 (d, 1H), 4.02 (qd, 1H), 3.94 (qd, 1H), 2,24 (s, 3H), 1.03 (t, 3H) |
| I-1-a-17 | F | 6-F | 3-EtO | Me | Bz | ¹H-NMR, 400MHz, d6-DMSO, 7.15-7.34 (m, 6H), 6.97-7.02 (m, 1H), 5.17 (s, 2H), 4.11 (q, 2H), 2,24 (s, 3H), 1.34 (t, 3H) |
| I-1-a-18 | F | H | 5-Cl | Me | Bz | ¹H-NMR, 400MHz, d6-DMSO, 7.41-7.45 (m, 1H), 7,24-7.37 (m, 7H), 5.16 (s, 2H), 2.23 (s, 3H) |
| I-1-a-19 | Cl | 6-Cl | H | c-Hexyl-CH₂ | Me | ¹H-NMR, 400MHz, d6-DMSO, 7.52 (d, 2H); 7.42 (dd, 1H); 3.58 (s, 3H); 2.50 (m, 2H, lösungsmittelüberlagert), 2.30 (m, 2H), 1.70 (m, 6H), 1.20 (m, 3H), 0.97 (m, 3H) |
| I-1-a-20 | Cl | 6-Cl | H | Me | 2-(4'-Chlorphenyl )-ethyl | ¹H-NMR, 400MHz, d6-DMSO, 10.8 (bs, 1H), 7.52 (d, 2H), 7.44 (dd, 1H); 7.30 (m, 2H), 7.18 (d, 2H); 4.22 (t, 2H); 2.98 (t, 2H), 2.21 (s, 3H), |
| I-1-a-21 | Cl | 4-Cl | H | Me | 2-(4'-Chlorphenyl )-ethyl | ¹H-NMR, 400MHz, d6-DMSO, 10.6 (bs, 1H), 7.70 (s, 1H), 7.47 (dd, 1H); 7.32 (m, 2H), 7.26 (d, 1H), 7.18 (d, 2H); 4.18 (m, 2H); 2.98 (t, 2H), 2.21 (s, 3H), |
| I-1 -a-22 | Cl | 4-Cl | H | Me | 2-(2'-Chlorphenyl )-ethyl | ¹H-NMR, 400MHz, d6-DMSO, 10.6 (bs, 1H), 7.70 (s, 1H), 7.47 (dd, 1H); 7.40 (m, 1H), 7.22 (m, 4H); 4.22 (m, 2H); 3.11 (t, 2H), 2.17 (s, 3H), |
| I-1-a-23 | F | 6-F | H | Me | Bz | ¹H-NMR, 400MHz, d6-DMSO, 7.43-7.51 (m, 1H); 7.23-7.33 (m, 6H); 7.06-7.12 (m, 1H); 5.18 (s, 2H); 2.25 (s, 3H) |
| I-1-a-24 | Cl | 6-F | 4-F | Me | Bz | Öl |
| I-1-a-25 | Cl | 6-F | H | Me | Bz | ¹H-NMR, 400MHz, d6-DMSO, 7.15-7.48 (m, 8H); 5.24 (d, 1H); 5.18 (d, 1H) ; 2.25 (s, 3H) |
| I-1-a-26 | Cl | 6-CF₃ | H | Me | 2-Phenylethyl | ¹H-NMR, 400MHz, CDCl₃, 7.68 (d, 2H), 7.47 (t, 1H); 7.21 (m, 5H); 6.20 (bs, 1H),4.28 (m, 2H); 3.06 (t, 2H), 2.28 (s, 3H), |
| I-1-a-27 | I | H | H | Me | 2-(4'-Chlorphenyl )-ethyl | ¹H-NMR, 400MHz, CDCI,, 7.99 (d, 2H), 7.47 (t, 1H); 7.20 (m, 6H); 5.60 (bs, 1H),4.32 (m, 2H); 3.08 (t, 2H), 2.32 (s, 3H), |
| I-1-a-28 | Cl | 4-Cl | 6-Et | c-Pentyl-CH₂I | Me | ¹H-NMR, 400MHz, d6-DMSO, 10.4 (bs, 1H), 7.52 (d, 1H), 7.37 (d, 1H); 3.58 (s, 3H), 2.58 (d, 2H); 2.31 (m, 2H), 2.22 (m, 1H), 1.8-1.2 (Signalhaufen, 8H), 1.03 (t, 3H) |
| I-1-a-29 | Cl | 6-Cl | H | c-Pentyl-CH₂ | Me | ¹H-NMR, 400MHz, d6-DMSO, 10.7 (bs, 1H), 7.52 (d, 2H), 7.41 (dd, 1H); 3.58 (s, 3H), 2.63 (d, 2H); 2.23 (m, 1H), 1.8 - 1.2 (Signalhaufen, 8H), |
| I-1-a-30 | Cl | 6-Cl | H | c-Pr | Me | ¹H-NMR, 400MHz, CDCl₃, 7.45 (d, 2H), 7.33 (t, 1H); 5.80 (bs, 1H),3.72 (s, 3H); 2.05 (m, 1H), 0.95 (m, 4H), |
| I-1-a-31 | H | 3-CF₃ | H | c-Pr-CH₂ | Me | |
| I-1-a-32 | Cl | 6-Cl | H | c-Pr-CH₂ | Me | |
| I-1-a-33 | Cl | 4-Cl | H | c-Pr-CH₂ | Me | |
| I-1-a-34 | H | 3-CF₃ | H | c-Pr-CH₂ | i-Pr | |
| I-1-a-35 | H | 2-Cl | H | c-Pr-CH₂ | i-Pr | |
| I-1-a-36 | Cl | 6-Cl | H | c-Pr-CH₂ | i-Pr | |
| I-1-a-37 | H | 2-Cl | H | 2-Phenylethyl | Me | |

### 3. Herstellung von 5-Acetoxy-6-cyclopentyl-4-(2,6-dichlorphenyl)-2, methyl-3(2H)-pyridazinon (Nr. 1 der Tabelle 92)

0.25 g (0.74 mmol) erfindungsgemäßer Verbindung I-1-a-5 der Tabelle 91 sowie 0.12 g Diisopropylethylamin (1.3eq) und eine Spatelspitze DMAP wurden in 15 ml Ethylacetat vorgelegt und unter Rückfluß zum Sieden erhitzt. Anschließend wurden 0.058 g (1.0 eq) Acetylchlorid in 2ml Ethylacetat gelöst und in 10 min zugetropft. Man ließ fünf Stunden rühren gab nochmals 0.04 g Diisopropylamin und 0.02 g Acetylchlorid hinzu, kühlte nach 2 weiteren Stunden Reaktionszeit ab und versetzte mit 20 ml Wasser und 50 ml Ethylacetat. Nach Abtrennung der organischen Phase und Trocknung erhielt man nach chromatographischer Reinigung (Silicagel, Gradient Ethylacetat /n-Heptan) 0.04 g an sauberem Produkt.
Analog der vorstehend genannten Methode sind die Verbindungen der Tabelle 92 erhältlich.

**Tabelle 92: Erfindungsgemäße Verbindungen der allgemeinen Formel (I), worin G für C(=O)R¹ steht.**

| | | | | | | | |
|---|---|---|---|---|---|---|---|
| 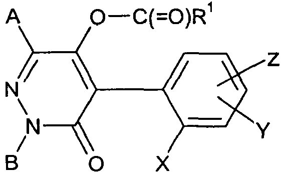 | | | | | | | |

| **Nr.** | **X** | **Y** | **Z** | **R¹** | **A** | **B** | **Analytische Daten** |
|---|---|---|---|---|---|---|---|
| I-1-b-1 | Cl | 6-Cl | H | Me | c-Pentyl | Me | ¹H-NMR, 400MHz, CDCl₃, 7.39 (d, 2H); 7.30 (d, 1H); 3.82 (s, 3H); 3.03 (m, 1H), 2.02 (s, 3H), 1.60 - 1.94 (Signalhaufen, 8H) |
| I-1-b-2 | Cl | 4-Cl | 6-Et | i-Pr | c-Pentyl | Me | ¹H-NMR, 400MHz, CDCl₃, 7.32 (d, 1H); 7.22 (d, 1H); 3.82 (s, 3H); 2.99 (m, 1H), 2.47 (m, 2H), 1.60-2.05 (Signalhaufen, 8H), 1.16 (t, 3H), 0.95 (dd, 6H) |

### 4. Herstellung von 4-(2,4-Dichlorophenyl)-5-ethoxycarbonyloxy-2-(2-Phenyl)-ethyl-3(2H)-pyridazinon (Nr. 1 der Tabelle 93)

1.25 g (0.61 mmol) der erfindungsgemäßen Verbindung Nr. I-1-a-7 der Tabelle 91 wurden in 30ml Dichlormethan vorgelegt und mit 0.438 g Triethylamin und 0.36 g Chlorameisensäureethylester versetzt. Man rührte über Nacht bei RT und gab anschließend auf 30ml fünfprozentige Natriumhydrogencarbonatlösung. Nach Abtrennung der organischen Phase wurde getrocknet, eingeengt und säulenchromatographisch (Silicagel, Gradient Ethylacetat/n-Heptan) gereinigt. Kristallisation aus Ethylacetat/n-Heptan lieferte 0.28 g an sauberem Produkt.

Analog der vorstehend genannten Methoden sind die Verbindungen der Tabelle 93 erhältlich.

**Tabelle 93: Erfindungsgemäße Verbindungen der allgemeinen Formel (I), worin G für C(=L)MR² steht.**

| | | | | | | | | | |
|---|---|---|---|---|---|---|---|---|---|
| 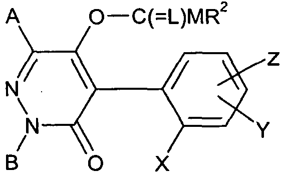 | | | | | | | | | |

| **Nr.** | **X** | **Y** | **Z** | **A** | **B** | **L** | **M** | **R²** | **Analytische Daten** |
|---|---|---|---|---|---|---|---|---|---|
| I-1-c-1 | Cl | 6-Cl | H | Me | 2-Phenylethyl | O | O | Et | ¹H-NMR, 400MHz, CDCl3, 7.39 (d, 2H); 7.18 - 7.32 (Signalhaufen, 6H); 4.42 (m, 2H); 4.17 (q, 2H), 3.15 (m, 2H), 2.31 (s, 3H), 1.22 (q, 3H) |
| I-1-c-2 | Cl | 4-Cl | H | Me | 2-Phenylethyl | O | O | Et | ¹H-NMR, 400MHz, CDCl3, 7.52 (d, 1H); 7.15 - 7.35 (Signalhaufen, 7H); 4.40 (m, 2H); 4.15 (q, 2H), 3.13 (m, 2H), 2.31 (s, 3H), 1.22 (q, 3H) |
| I-1-c-3 | Cl | 6-Cl | H | Me | 2-(2'-Cl-Phenylethyl | O | O | Et | ¹H-NMR, 400MHz, CDCl3, 7.10-7.40 (Signalhaufen, 7H); 4.48 (m, 2H); 4.18 (m, 2H), 3.28 (m, 2H), 2.29 (s, 3H), 1.21 (q, 3H) |
| I-1-c-4 | Cl | 6-Et | 4-Cl | Me | c-Pr-CH₂ | O | O | Et | ¹H-NMR, 400MHz, d6-DMSO, 7.60 (d, 1H); 7.43 (d, 1H); 4.13 (q, 2H), 3.99 (d, 2H); 2.35 (m, 2H), 2.28 (s, 3H), 1.22 (m, 1H), 1,08 (t, 3H), 1.03 (t ,3H), 0.48 (m, 2H), 0.36 (m, 2H) |
| I-1-c-5 | Cl | 6-Cl | H | c-Pentyl | Me | O | O | Et | ¹H-NMR, 400MHz, CDCl3). 7.39 (d, 2H); 7.29 (d, 1H); 4.11 8q, 2H), 3.83 (s, 3H); 3.12 (m, 1H), 1.96 (m, 2H), 1.82 (m, 4H), 1.65 (m ,2H), 1.13 (t, 3H) |
| I-1-c-6 | Cl | 6-Et | 4-Cl | c-Pentyl | Me | O | O | Et | ¹H-NMR, 400MHz, CDCl3 7.32 (d, 1H); 7.22 (d, 1H); 4.08 (q, 2H), 3.83 (s, 3H); 3.08 (m, 1H), 2.47 (m, 2H), 1.98 (m, 2H), 1.80 (m, 4H), 1.65 (m ,2H), 1.15 (m, 6H) |
| I-1-c-7 | Cl | 6-Cl | H | Me | c-Pr-CH₂ | O | O | Et | ¹H-NMR, 400MHz, d6-DMSO, 7.62 (m, 2H); 7.52 (t, 1H); 4.36 (m, 1H), 4.13 (m, 2H), 3.99 (m, 1H); 2.28 (s, 3H), 1.30 - 1.00 (Signalhaufen, 4H), 0.52 (m, 2H), 0.40 (m, 2H) |
| I-1-c-8 | I | H | H | Me | 2-Phenylethyl | O | O | Et | ¹H-NMR, 400MHz, CDCl3 7.92 (d, 1H); 7.39 (t, 1H); 7.30 - 7.05 (Signalhaufen, 7H), 4.40 (m, 2H), 4.13 (q, 2H), 3.13 (t, 2H), 2.30 (s, 3H), 1.18 (m, 6H) |
| I-1-c-9 | I | H | H | Me | 2-(2'-Cl-Phenylethyl | O | O | Et | ¹H-NMR, 400MHz, CDCl3 7.92 (d, 1H); 7.35 (m, 2H); 7.27 8m, 1H), 7.15 (m, 3H), 7.08 (m, 1H), 4.48 (m, 2H), 4.13 (q, 2H), 3.31 (m, 2H), 2.28 (s, 3H), 1.17 (m, 6H) |
| I-1-c-10 | Cl | 6-Cl | H | CF₃(CH₂)₂ | Me | O | O | Et | ¹H-NMR, 400MHz, d6- CDCl₃, 7.42 (m, 2H); 7.32 (t, 1H); 4.36 (m, 1H), 4.13 (m, 2H), 3.85 (s, 3H); 2.88 (t, 2H), 2.62 (m, 2H), 1.18 (t, 3H) |
| I-1-c-11 | Cl | 6-Et | 4-Cl | c-Hexyl-CH₂ | Me | O | O | Et | ¹H-NMR, 400MHz, CDCl₃ 7.32 (d, 1H); 7.22 (d, 1H); 4.12 (q, 2H), 3.83 (s, 3H); 2.48 (d, 2H), 2.45 (m, 2H), 1.70 (m, 6H), 1.40 - 0.90 (Signalhaufen, 9H), |
| I-1-c-12 | Cl | 6-Cl | H | c-Hexyl-CH₂ | Me | O | O | Et | ¹H-NMR, 400MHz, CDCl3), 7.39 (d, 2H); 7.29 (d, 1H); 4.11 (q, 2H), 3.83 (s, 3H); 2.48 (d, 2H), 1.70 (m, 6H), 1.20 (m, 2H), 1.15 (t, 3H), 0.99 (m, 2H), |
| I-1-c-13 | Cl | 6-Cl | H | Me | 2-(4'-Cl-phenyl)-ethyl | O | O | Et | ¹H-NMR, 400MHz, CDCl3), 7.40 (d, 2H); 7.28 (d, 1H); 4.41 (t, 2H), 4.16 (q, 2H), 3.11 (m, 2H), 2.32 (s, 3H), 1.22 (t, 3H) |
| I-1-c-14 | Cl | 4-Cl | H | Me | 2-(4'Cl-phenyl)-ethyl | O | O | Et | ¹H-NMR, 400MHz, CDCl3), 7.51 (s, 1H); 7.32 (d, 1H); 7.27 (d, 2H), 7.18 (m, 3H), 4.38 (m, 2H), 4.18 (q, 2H), 3.13.(m, 2H), 2.30 (s, 3H), 1.24 (t, 3H) |
| I-1-c-15 | Cl | 4-Cl | H | Me | 2-(2'-Cl-phenyl)-ethyl | O | O | Et | ¹H-NMR, 400MHz, CDCl3), 7.51 (s, 1H); 7.35 (m, 1H); 7.32 (d, 1H), 7.22 (m, 1H), 7.16 (m, 3H), 4.43 (m, 2H), 4.18 (q, 2H), 3.28.(m, 2H), 2.27 (s, 3H), 1.24 (t, 3H) |
| I-1-c-16 | Cl | 6-CF₃ | H | Me | 2-Phenylethyl | O | O | Et | ¹H-NMR, 400MHz, CDCl3), 7.68 (d, 2H); 7.51 (t, 1H); 7.25 (Signalhaufen, 5H), 4.42 (m, 2H), 4.18 (q, 2H), 3.13.(m, 2H), 2.29 (s, 3H), 1.24 (t, 3H) |
| I-1-c-17 | I | H | H | Me | 2-(4'-Cl-phenyl)-ethyl | O | O | Et | ¹H-NMR, 400MHz, CDCl3 7.92 (d, 1H); 7.39 (t, 1H); 7.20 (d, 2H), 7.12 (m, 2H), 4.38 (m, 2H), 4.16 (q, 2H), 3.12 (t, 2H), 2.31 (s, 3H), 1.18 (m, 3H) |
| I-1-c-18 | Cl | 6-Et | 4-Cl | c-Pentyl-CH₂ | Me | O | O | Et | ¹H-NMR, 400MHz, CDCl3 7.32 (d, 1H); 7.21 (d, 1H); 4.12 (m, 2H), 3.82 (s, 3H); 2.62 (d, 2H), 2.45 (m, 2H), 2.28 (m, 1H), 1.70 (m, 4H), 1.40-1.10 (Signalhaufen, 10H), |
| I-1-c-19 | Cl | 6-Cl | H | c-Pentyl-CH₂ | Me | O | O | Et | ¹H-NMR, 400MHz, CDCl3), 7.39 (d, 2H); 7.29 (d, 1H); 4.11 (q, 2H), 3.83 (s, 3H); 2.62 (d, 2H), 2.45 (m, 2H), 2.28 (m, 1H), 1.70 (m, 4H), 1.55 (m, 1H), 1.40 - 1.10 (Signalhaufen, 5H), |
| I-1-c-20 | Cl | 4-Cl | H | c-Pentyl-CH₂ | Me | O | O | Et | ¹H-NMR, 400MHz, CDCl3), 7.49 (d, 1H); 7.32 (dd, 1H), 7.18 (d, 1H); 4.12 (q, 2H), 3.83 (s, 3H); 2.62 (d, 2H), 2.45 (m, 2H), 2.28 (m, 1H), 1.70 (m, 4H), 1.55 (m, 1H), 1.40 - 1.10 (Signalhaufen, 5H), |
| I-1-c-21 | I | H | H | Me | 2-(4'-Cl-phenyl)-ethyl | O | O | Me | ¹H-NMR, 400MHz, CDCl3 7.93 (d, 1H); 7.41 (t, 1H); 7.35 -7.20 (Signalhaufen, 5 H), 7.16 (d, 2H), 7.10 (m, 2H), 4.42 (m, 2H), 3.75 (s, 3H), 3.14 (t, 2H), 2.31 (s, 3H) |
| I-1-c-22 | Cl | 6-Cl | H | c-Pr | Me | O | O | Et | ¹H-NMR, 400MHz, CDCl₃, 7.39 (d, 2H), 7.28 (t, 1H); 4.17 (q, 2H),3.78 (s, 3H); 1.89 (m, 1H), 1.22 8t, 3H), 1.05 (m, 2H), 0.97 (m, 2H) |

### 5. Herstellung von 4-(2,lodophenyl)-5-methylsulfonyloxy-2-(2-(2'-Chlorphenyl)-ethyl-3(2H)-pyridazinon (Nr. 1 der Tabelle 93)

0.14 g (0.3 mmol) der erfindungsgemäßen Verbindung Nr. I-1-a-10 der Tabelle 91 wurden in 10ml Dichlormethan vorgelegt und mit 0.039 g Triethylamin und 0.034 g Methansulfonsäurechlorid versetzt. Man rührte über Nacht bei RT und gab anschließend auf 5ml Wasser. Nach Abtrennung der organischen Phase wurde getrocknet, eingeengt und säulenchromatographisch (Silicagel, Gradient Ethylacetat/n-Heptan) gereinigt. Kristallisation aus Ethylacetat/n-Heptan lieferte 0.15 g an sauberem Produkt.

**Tabelle 94: Erfindungsgemäße Verbindungen der allgemeinen Formel (I)**

| | | | | | | | |
|---|---|---|---|---|---|---|---|
| 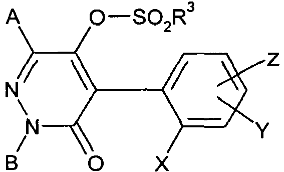 | | | | | | | |

| **Nr.** | **X** | **Y** | **Z** | **R³** | **A** | **B** | **Analytische Daten** |
|---|---|---|---|---|---|---|---|
| I-1-b-1 | I | H | H | Me | Me | 2-(4'-Cl-phenyl)-ethyl | ¹H-NMR, 400MHz, CDCl3 7.92 (d, 1H); 7.46 (m, 1H); 7.33 (m, 1H), 7.28 (m, 1H), 7.20 (m, 1H)7.15 (m, 3H), , 4.48 (m, 2H), 3.28 (m, 2H), 2.46 (s, 3H), 2.44 (s, 3H) |

### 6. beispielhafte Herstellung von Verbindungen der Formel (IIa):

3.22 g (26.4 mmol) Benzylhydrazin wurden zusammen mit 4.4 ml (1.2 eq) Triethylamin und 0.16 g DMAP (0.05eq) in 50 ml Dichlormethan vorgelegt und anschließend 5.5 g 2,3,6-Trifluorophenylessigsäurechlorid in 50 ml Dichlormethan bei 0 °C langsam zugetropft. Anschließend wurde über Nacht bei Raumtemperatur gerührt und mit Ammoniumchloridlösung versetzt. Die organische Phase wurde abgetrennt, getrocknet und eingeengt. Nach säulenchromatographischer Reinigung erhielt man 6.2 g der 2,3,6-Trifluorophenylessigsäure-1-methylhydrazid.

### Herstellung von Verbindungen der Formel (II)

Beispiel 1: 1 g 2,3,6-Trifluorophenylessigsäure-1-methylhydrazid wurde mit 395 mg Ethylpyruvat versetzt und in 10 ml Ethanol gelöst. Man erhitzte 6 h unter Rückfluss zum Sieden, engte das Reaktionsgemisch anschließend unter vermindertem Druck ein und reinigte es säulenchromatographisch (Kieselgel, Eluent Cyclohexan/Ethylacetat 4:1). Man erhielt 1.03 g der Verbindung 1 der Tabelle 95.

**Tabelle 95: Erfindungsgemäße Verbindungen der allgemeinen Formel (II)**

| | | | | | | | |
|---|---|---|---|---|---|---|---|
| 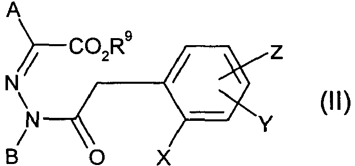 | | | | | | | |

| **Nr.** | **X** | **Y** | **Z** | **A** | **B** | **R⁹** | **Analytische Daten** |
|---|---|---|---|---|---|---|---|
| II-a-1 | F | 3-F | 6-F | Me | Bz | Et | ¹H NMR (400 MHz, d6-DMSO, Shift in ppm) : 7.62 (s, 2H); 7.24-7.36 (m, 5H); 5.09 (s, 2H); 4.20-4.25 (m, 4H); 2.05 (bs, 3H), 1.25(t, 3H) |
| II-a-2 | Br | 4-Br | 6-Me | Me | c-Pr-CH₂ | Me | ¹H NMR (400 MHz, d6-DMSO, Shift in ppm) : 7.68 (s, 1H); 7.46 (s, 1H); 3.97 (d, 2H); 3.82 (s, 3H); 3.75 (s, 2H), 2.28 (s, 3H), 2.30 und 1.92 (je s, zusammen 3H), 1.05 (m, 1H), 0.60 - 0.10 (Signalhaufen, 4H) |
| II-a-3 | Cl | 6-Cl | 4-Cl | Me | Bzl | Et | ¹H NMR (400 MHz, d6-DMSO, Shift in ppm) :7.62 (s, 2H); 7.24-7.36 (m, 5H); 5.09 (s, 2H); 4.20-4.25 (m, 4H); 2.05 (bs, 3H), 1.25(t, 3H) |
| II-a-4 | Cl | 3-Me | 6-F | Me | Bz | Et | ¹H NMR (400 MHz, d6-DMSO, Shift in ppm) :7.17-7.36 (m, 7H); 5.09 (s, 2H); 4.22 (q, 2H); 4.08 (s, 2H); 2.21 (s, 3H), 2.02 (bs, 3H); 1.25(t, 3H) |
| II-a-5 | Cl | 6-Cl | H | Me | c-Pr-CH₂ | Me | ¹H NMR (400 MHz, d6-DMSO, Shift in ppm) : 7.48 (d, 2H); 7.32 (t, 1H); 4.12 (d, 2H); 3.82 (s, 3H); 3.75 (s, 2H), 2.30 und 1.88 (je s, zusammen 3H), 1.05 (m, 1H), 0.60 - 0.10 (Signalhaufen, 4H) |
| II-a-6 | Cl | 4-Me | 6-Me | Me | c-Pr-CH₂ | Me | ¹H NMR (400 MHz, d6-DMSO, Shift in ppm): 7.10 (s, 1H); 6.98 (s, 1H); 3.92 (d, 2H); 3.82 (s, 3H); 3.75 (s, 2H), 2.28 (s, 3H), 2.20 (s, 3H), 2.30 und 1.92 (je s, zusammen 3H), 1.05 (m, 1H), 0.60 - 0.10 (Signalhaufen, 4H) |
| II-a-7 | Cl | 4-Cl | 6-Et | c-pentyl | Me | Et | ¹H NMR (400 MHz, CDCl₃, Shift in ppm): 7.27 (s, 1H); 7.10 (s, 1H); 4.32 (m, 2H); 4.12 (s, 2H), 3.32 (s, 3H); 2.98 (s, 1H), 2.58 (m, 2H), 1.92 (m, 2H), 1.85 -1.60 (Signalhaufen, 6H), 1.37 (t, 3H), 1.18 (t, 3H) |
| II-a-8 | Cl | 6-Cl | H | c-pentyl | Me | Et | ¹H NMR (400 MHz, CDCl₃, Shift in ppm) : 7.32 (d, 2H); 7.15 (t, 1H); 4.32 (m, 4H); 3.23 (s, 3H); 2.96 (s, 1H), 1.92 (m, 2H), 1.85 -1.60 (Signalhaufen, 6H), 1.37 (t, 3H) |
| II-a-9 | Cl | 6-Cl | H | Me | 2-Phenylethyl | Me | ¹H NMR (400 MHz, CDCl₃, Shift in ppm) : 7.35 - 7.10 (Signalhaufen, 8 H), 4.26 und 3.96 (je s, zusammen 2H), 4.22 und 4.10 (je t, zusammen 2H), 3.89 (s, 3H), 2.96 (m, 2H), 2.32 und 1.98 (je s, zusammen 3H) |
| II-a-10 | Cl | 6-Cl | H | Me | 2-(2'-Cl-phenyl)-ethyl | Me | ¹H NMR (400 MHz, CDCl₃, Shift in ppm) : 7.35 - 7.10 (Signalhaufen, 7 H), 4.22 und 3.98 (je s, zusammen 2H), 4.22 und 4.10 (je m, zusammen 2H), 3.89 (d, 3H), 3.12 (m, 2H), 2.32 und 1.93 (je s, zusammen 3H) |
| II-a-11 | I | H | H | Me | 2-Phenylethyl | Me | ¹H NMR (400 MHz, CDCl₃, Shift in ppm) : 7.82 (d, 1H), 7.35 - 7.10 (Signalhaufen, 7 H), 6.93 (t, 1H), 4.06 und 3.71 (je m, zusammen 4H), 3.89 (s, 3H), 2.92 (m, 2H), 2.25 und 1.95 (je s, zusammen 3H) |
| II-a-12 | I | H | H | Me | 2-(2'-Cl-phenyl)-ethyl | Me | ¹H NMR (400 MHz, CDCl₃, Shift in ppm) : 7.82 (d, 1H), 7.35 - 7.10 (Signalhaufen, 6 H), 6.93 (t, 1H), 4.08 und 3.76 (je m, zusammen 4H), 3.89 (s, 3H), 3.09 (m, 2H), 2.27 und 1.98 (je s, zusammen 3H) |
| II-a-13 | F | 6-Cl | 4-F | Me | Bz | Et | ¹H NMR (400 MHz, d6-DMSO, Shift in ppm) :7.22-7.36 (m, 7H); 5.07 (s, 2H); 4.22 (q, 2H); 4.04 (s, 2H); 2.02 (bs, 3H); 1.25 (t, 3H) |
| II-a-14 | F | 6-F | H | Me | Bzl | Et | ¹H-NMR (400MHz, CD₃CN, Shift in ppm): 7.21-7.38 (m, 7H); 6.91-6.98 (m, 1H); 5.18 (s, 2H); 4.24 (q, 2H); 3.98 (s, 2H); 2.08 (bs, 3H); 1.28 (t, 3H) |
| II-a-15 | F | 5-F | H | Me | Bz | Et | ¹H NMR (400 MHz, d6-DMSO, Shift in ppm) :7.16-7.44 (m, 8H), 5.06 (s, 1H), 4.23 (q, 2H), 3.99 (s, 2H), 2.00 (broad s, 3H), 1.25 (t, 3H) |
| II-a-16 | Cl | 6-Et | 4-Cl | Me | Bz | Me | ¹H-NMR (400MHz, d6-DMSO, Shift in ppm): 7.50-7.10 (m, 6H); 7.08 (m, 1H); 5.12 (s, 2H); 4.13 (s, 2H); 3.78 8s, 3H), 2.56 (m, 2H), 2.13 und 1.85 (je s, zusammen 3H) 1.14 und 1.02 (je m, zusammen 3H) |
| II-a-17 | Cl | 6-Et | 4-Cl | Me | c-Pr-CH₂ | Me | ¹H-NMR (400MHz, d6-DMSO, Shift in ppm): 7.42 (s, 1H); 7.28 (s, 1H); 5.12 (s, 2H); 3.98 (d, 2H); 3.80 (s, 3H); 3.77 (s, 2H), 2.28 und 1.88 (je s, zusammen 3H), 1.11 (t, 3H), 1.05 (m, 1H), 0.60 - 0.10 (Signalhaufen, 4H) |
| II-a-18 | Cl | 4-Cl | H | Me | c-Butyl | Me | ¹H-NMR (400MHz, d6-DMSO, Shift in ppm): 7.58 (s, 1H); 7.38 (s, 2H); 4,85 (s, 1H); 3.95 (s, 2H); 2,26 (m, 2H); 2.22 - 1.70 (Signalhaufen, 5H), 1.65 (m, 2H) |
| II-a-19 | Cl | 6-Et | 4-Cl | c-hexyl-CH₂ | Me | Et | ¹H-NMR (400MHz, CDCl₃, Shift in ppm): 7.28 (s, 1H), 7.11 (s, 1H), 4.32 (q, 2H), 4.11 (s, 2H), 3.32 (s, 3H), 2.58 (d, 2H), 2.42 (q, 2H), 1.72 (m, 6H), 1.38 (t, 3H), 1.20 (m, 7H), 1.02 (m, 2H) |
| II-a-20 | Cl | 6-Cl | H | c-hexyl-CH₂ | Me | Et | ¹H-NMR (400MHz, CDCl₃, Shift in ppm): 7.32 (dd, 2H); 7.14 (m, 1H); 4,32 (m, 4H); 3.40 und 3.21 (je s, zusammen 3H); 2.58 und 2.42 (je d, zusammen 2H); 1.90 - 1.60 (Signalhaufen, 7H), 1.39 (m, 3H), 1.35 - 0.95 (Signalhaufen, 4H) |
| II-a-21 | Cl | 6-Cl | H | Me | 2-(4'-Cl-phenyl)-ethyl | Me | ¹H-NMR (400MHz, CDCl₃, Shift in ppm): 7.30 (m, 4H); 7.17 (m, 3H); 4,28 - 3.80 (Signalhaufen 7H); 2.92 (m, 2H); 2.30 und 1.92 (je s, zusammen 3H) |
| II-a-22 | Cl | 4-Cl | H | Me | 2-(4'-Cl-phenyl)-ethyl | Me | ¹H-NMR (400MHz, CDCl₃, Shift in ppm): 7.38 (d, 1H); 7.26 (d, 2H), 7.19 (dd, 1H), 7.13 (m, 3H); 4.05 (m, 2H), 3.93 und 3.62 (je s, zusammen 2H), 3.88 (s, 3H), 2.85 (m, 2H); 2.30 und 1.92 (je s, zusammen 3H) |
| II-a-23 | Cl | 4-Cl | H | Me | 2-(2'-Cl-phenyl)-ethyl | Me | ¹H-NMR (400MHz, CDCl₃, Shift in ppm): 7.42 - 7.11 (Signalhaufen, 7H); 4.12 (m, 2H), 3.98 und 3.62 (je s, zusammen 2H), 3.88 (s, 3H), 3.08 (m, 2H); 2.28 und 1.92 (je s, zusammen 3H) |
| II-a-24 | Cl | 6-CF₃ | H | Me | 2-Phenylethyl | Me | ¹H-NMR (400MHz, CDCl₃, Shift in ppm): 7.62 (m, 2H); 7.32 (m, 3H), 7.20 (m, 3H), 4.12 (m, 2H), 4.22 und 3.98 (je s, zusammen 2H), 3.91 (s, 3H), 3.02 und 2.88 (je t, zusammen 2H); 2.33 und 1.85 (je s, zusammen 3H) |
| II-a-25 | I | H | H | Me | 2-(4'-Cl-phenyl)-ethyl | Me | ¹H-NMR (400MHz, CDCl₃, Shift in ppm): 7.82 (d, 1H); 7.30 (m, 3H), 7.15 (m, 3H), 6.96 (m, 1H); 4.01 (m, 2H), 3.98 und 3.62 (je s, zusammen 2H), 3.90 (s, 3H), 2.88 (m, 2H); 2.24 und 1.96 (je s, zusammen 3H) |
| II-a-26 | Cl | 4-Cl | 6-Et | c-pentyl-CH₂ | Me | Et | ¹H-NMR (400MHz, CDCl₃, Shift in ppm): 7.29 (d, 1H); 7.12 (d, 1H), 4.32 (m, 2H), 4.12 (d, 2H), 3.38 und 3.22 (je s, zusammen 3H), 2.58 (m, 4H), 1.90 - 1.60 (Signalhaufen, 6H), 1.37 und 1.22 (je m, zusammen 9H) |
| II-a-27 | Cl | 6-Cl | H | c-pentyl-CH₂ | Me | Et | ¹H-NMR (400MHz, CDCl₃, Shift in ppm): 7.33 (d, 2H); 7.15 (m, 1H), 4.32 (m, 4H), 3.40 und 3.22 (je s, zusammen 3H), 2.80 und 2.55 (je d, zusammen 2H), 1.90 -1.60 (Signalhaufen, 7H), 1.38 (m, 3H), und 1.20 (m, 2H) |
| II-a-28 | Cl | 4-Cl | H | c-pentyl-CH₂ | Me | Et | ¹H-NMR (400MHz, CDCl₃, Shift in ppm): 7.38 (s, 1H); 7.22 (m, 2H), 4.32 (q, 2H), 4.08 (s, 2H), 3.38 (s, 3H), 2.72 (d, 2H), 2.00 (m, 1H),1.68 8m, 4H), 1.53 (m, 2H), 1.38 (t, 3H), und 1.10 (m, 2H) |
| II-a-29 | Cl | 6-Cl | H | c-Pr | Me | Et | ¹H-NMR (400MHz, CDCl₃, Shift in ppm): 7.31 (d, 2H); 7.12 (m, 1H), 4.31 (m, 2H), 4.22 (s, 2H), 3.48 (s, 3H), 1.90 (m, 1H), 1.36 (t, 3H), und 1.12 (m, 4H) |
| II-a-30 | Cl | Cl | H | CF₃(CH₂)₂ | Me | Et | ¹H-NMR (400MHz, CDCl₃, Shift in ppm): 7.33 (d, 2H); 7.15 (m, 1H), 4.32 (m, 4H), 3.50 und 3.28 (je s, zusammen 3H), 3.15 und 3.02 (je m, zusammen 2H), 2.82 und 2.55 (je d, zusammen 2H), 1.38 (m, 3H) |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (@Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 I/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. I-1-c-4 und I I-1-c-7 bei einer Aufwandmenge von 320 g/ha jeweils eine mindestens 90%-ige Wirkung gegen Echinochloa crus galli, Stellaria media und Veronica persica.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 I/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigt beispielsweise die Verbindung Nr. I-1-c-4 bei einer Aufwandmenge von 80 g/ha jeweils eine mindestens 80%-ige Wirkung gegen Abutilon theophrasti, Alopecurus myosuroides, Avena fatua, Echinochloa crus galli, Lolium multiflorum, Setaria viridis und Veronica persica.

### 3. Insektizide Wirkung

### Beispiel A

### Myzus-Test (MYZUPE Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (Brassica pekinensis), die von allen Stadien der Grünen Pfirsichblattlaus (Myzus persicae) befallen sind, werden mit einer Wirkstoffzubereitung der gewünschten Konzentration gespritzt. Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden. Bei diesem Test zeigt z. B. die Verbindung Nr. I-1-a-4 eine mindestens 80%-ige Wirkung bei einer Aufwandmenge von 500 g / ha.

### Beispiel B

### Spodoptera frugiperda-Test (SPODFR Spritzbehandlung)

| | |
|---|---|
| Lösungsmittel: | 78,0 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Maisblattscheiben (Zea mays) werden mit einer Wirkstoffzubereitung der gewünschten Konzentration gespritzt und nach dem Abtrocknen mit Raupen des Heerwurms (Spodoptera frugiperda) besetzt. Nach der gewünschten Zeit wird die Wirkung in % bestimmt. Dabei bedeutet 100 %, dass alle Raupen abgetötet wurden; 0 % bedeutet, dass keine Raupe abgetötet wurde. Bei diesem Test zeigt z. B. die Verbindung Nr. I-1-a-9 eine mindestens 80%-ige Wirkung bei einer Aufwandmenge von 500 g / ha.

### Beispiel C

### Meloidogyne incognita-Test (MELGIN)

| | |
|---|---|
| Lösungsmittel: | 78,0 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Gefäße werden mit Sand, Wirkstofflösung, Meloidogyne incognita-Ei-Larvensuspension und Salatsamen gefüllt. Die Salatsamen keimen und die Pflänzchen entwickeln sich. An den Wurzeln entwickeln sich die Gallen. Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht Bei diesem Test zeigen z. B. die Verbindungen Nr. I-1-a-9 und I-1-a-10 eine mindestens 80%-ige Wirkung bei einer Aufwandmenge von 20 ppm.

## Patentansprüche

1. 4-Phenylpyridazinone der Formel (I) oder deren Salze 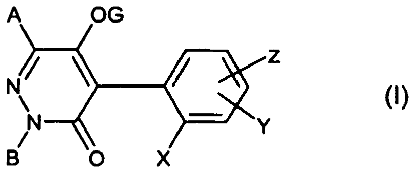 worin
A bedeutet unabhängig voneinander jeweils Wasserstoff, (C₃-C₆)-Cycloalkyl oder durch n Reste aus der Gruppe bestehend aus Halogen, (C₃-C₆)-Cycloalkyl, Phenyl und Halogenphenyl substituiertes (C₁-C₆)-Alkyl,
B bedeutet unabhängig voneinander (C₃-C₆)-Cycloalkyl, oder durch m Reste aus der Gruppe bestehend aus Halogen, (C₃-C₆)-Cycloalkyl, Phenyl und Halogenphenyl substituiertes (C₁-C₆)-Alkyl;
m bedeutet 0, 1, 2 oder 3;
n bedeutet 0, 1, 2 oder 3, mit der Maßgabe, dass m und n nicht gleichzeitig 0 bedeuten;
G bedeutet Wasserstoff, C(=O)R¹, C(=L)MR², SO₂R³, P(=L)R⁴R⁵, C(=L)NR⁶R⁷, E;
E bedeutet ein Metallionäquivalent oder ein Ammonium;
L bedeutet Sauerstoff oder Schwefel;
M bedeutet Sauerstoff oder Schwefel;
R¹ bedeutet jeweils durch n Halogenatome substituiertes (C₁-C₆)-Alkyl, (C₂-C₆)-Alkenyl, (C₁-C₄)-Alkoxy-(C₁-C₆)-alkyl, Di-(C₁-C₄)-alkoxy-(C₁-C₆)-alkyl oder (C₁-C₄)-Alkylthio-(C₁-C₆)-alkyl,
einen durch n Reste aus der Gruppe bestehend aus Halogen, (C₁-C₄)-Alkyl und (C₁-C₄)-Alkoxy substituiertes (C₃-C₆)-Cycloalkyl, Phenyl oder Phenyl-(C₁-C₄)-alkyl;
R² bedeutet durch n Halogenatome substituiertes (C₁-C₆)-Alkyl oder jeweils durch n Reste aus der Gruppe bestehend aus Halogen, (C₁-C₄)-Alkyl und (C₁-C₄)-Alkoxy substituiertes (C₃-C₆)-Cycloalkyl, Phenyl oder Benzyl;
R³, R⁴ und R⁵ bedeuten unabhängig voneinander jeweils durch n Halogenatome substituiertes (C₁-C₆)-Alkyl oder durch n Reste aus der Gruppe bestehend aus Halogen, (C₁-C₄)-Alkyl und (C₁-C₄)-Alkoxy substituiertes Phenyl oder Benzyl;
R⁶ und R⁷ bedeuten unabhängig voneinander jeweils Wasserstoff, durch n Halogenatome substituiertes (C₁-C₆)-Alkyl oder durch n Reste aus der Gruppe bestehend aus Halogen, (C₁-C₄)-Alkyl und (C₁-C₄)-Alkoxy substituiertes Phenyl oder Benzyl;
und
a)
X bedeutet Wasserstoff, Methyl, Ethyl oder cyclo-Propyl;
Y bedeutet Halogen, Cyano, Nitro, Halogen-(C₁-C₆)-alkyl, Halogen-(C₁-C₆)-alkoxy, (C₃-C₆)-Cycloalkyl, (C₁-C₆)-Alkoxy oder durch n Halogenatome substituiertes Phenyl,
Z bedeutet Wasserstoff, Halogen, Halogen-(C₁-C₆)-alkyl, Halogen-(C₁-C₆)-alkoxy, (C₃-C₆)-Cycloalkyl oder durch n Halogenatome substituiertes Phenyl,
oder
b)
X bedeutet Halogen, Cyano, Nitro, Halogen-(C₁-C₆)-alkyl, Halogen-(C₁-C₆)-alkoxy, (C₁-C₆)-Alkoxy oder (C₃-C₆)-Cycloalkyl;
Y bedeutet Wasserstoff, Halogen, Cyano, Nitro, (C₃-C₆)-Cycloalkyl, durch jeweils n Halogenatome substituiertes (C₁-C₆)-Alkyl, (C₁-C₆)-Alkoxy oder Phenyl;
Z bedeutet Wasserstoff, Halogen, Cyano, Nitro, (C₃-C₆)-Cycloalkyl oder durch jeweils n Halogenatome substituiertes (C₁-C₆)-Alkyl, (C₁-C₆)-Alkoxy oder Phenyl.

2. 4-Phenylpyridazinone nach Anspruch 1, worin
A bedeutet cyclo-Propyl, cyclo-Propylmethyl, Benzyl, 2-Chlorphenylmethyl, 3-Chlorphenylmethyl oder 4-Chlorphenylmethyl;
B bedeutet Wasserstoff, 2,2-Difluoroethyl, 2,2,2-trifluoroethyl, cyclo-Propyl, cyclo-Propylmethyl, Benzyl, 2-Chlor-Phenylmethyl, 3-Chlor-Phenylmethyl oder 4-Chlor-Phenylmethyl;
G bedeutet Wasserstoff, C(=O)R¹, C(=L)MR², SO₂R³, P(=L)R⁴R⁵, C(=L)NR⁶R⁷, E;
E bedeutet Na⁺, K⁺, (Mg²⁺)_{1/2}, (Ca²⁺)_{1/2}, (CH₃)₄N⁺ oder NH₄⁺;
L bedeutet Sauerstoff;
M bedeutet Sauerstoff;
R¹ bedeutet (C₁-C₆)-Alkyl oder (C₃-C₆)-Cycloalkyl;
R² bedeutet (C₁-C₆)-Alkyl, (C₃-C₆)-Cycloalkyl oder Benzyl;
R³, R⁴ und R⁵ bedeuten unabhängig voneinander jeweils (C₁-C₆)-Alkyl, Phenyl oder Benzyl;
R⁶ und R⁷ bedeuten unabhängig voneinander jeweils Wasserstoff, (C₁-C₆)-Alkyl, Phenyl oder Benzyl;
und
a)
X bedeutet Wasserstoff, Methyl oder Ethyl;
Y bedeutet Fluor, Brom, Chlor, Iod, Cyano, Nitro, cyclo-Propyl, Methoxy, Ethoxy, Trifluormethyl oder Trifluormethoxy, Phenyl oder Halogenphenyl;
Z bedeutet Wasserstoff, Fluor, Brom, Chlor, Iod, Methyl, Ethyl, Methoxy, Ethoxy, Trifluormethyl oder cyclo-Propyl;
oder
b)
X bedeutet Fluor, Brom, Chlor, Iod, Cyano, Nitro, Methoxy, Ethoxy, Trifluormethyl, Trifluormethoxy oder cyclo-Propyl
Y bedeutet Wasserstoff, Fluor, Brom, Chlor, Iod, Methyl, Ethyl, Methoxy, Ethoxy, Trifluormethyl, Trifluormethoxy oder cyclo-Propyl;
Z bedeutet Wasserstoff, Fluor, Brom, Chlor, Iod, Methyl, Ethyl, Methoxy, Ethoxy, Trifluormethyl, Trifluormethoxy, cyclo-Propyl, Chlorphenyl oder Fluorphenyl.

3. Herbizide Mittel, **gekennzeichnet durch** einen herbizid wirksamen Gehalt an mindestens eines 4-Phenylpyridazinons der Formel (I) gemäß Anspruch 1 oder 2.

4. Herbizide Mittel nach Anspruch 3 in Mischung mit Formulierungshilfsmitteln.

5. Herbizide Mittel nach Anspruch 3 oder 4 enthaltend mindestens einen weiteren pestizid wirksame Stoffen aus der Gruppe Insektizide, Akarizide, Herbizide, Fungizide, Safenem und Wachstumsregulatoren.

6. Herbizide Mittel nach Anspruch 5 enthaltend einen Safener.

7. Herbizide Mittel nach Anspruch 5 oder 6 enthaltend ein weiteres Herbizid.

8. Verfahren zur Bekämpfung unerwünschter Pflanzen, **dadurch gekennzeichnet, daß** man eine wirksame Menge mindestens eines 4-Phenylpyridazinons der Formel (I) gemäß Anspruch 1 oder 2 oder eines herbiziden Mittels nach einem der Ansprüche 3 bis 7 auf die Pflanzen oder auf den Ort des unerwünschten Pflanzenwachstums appliziert.

9. Verwendung von 4-Phenylpyridazinonen der Formel (I) gemäß Anspruch 1 oder 2 oder von herbiziden Mitteln nach einem der Ansprüche 3 bis 4 zur Bekämpfung unerwünschter Pflanzen.

10. Verwendung nach Anspruch 9, **dadurch gekennzeichnet, daß** die 4-Phenylpyridazinone der Formel (I) zur Bekämpfung unerwünschter Pflanzen in Kulturen von Nutzpflanzen eingesetzt werden.

11. Verwendung nach Anspruch 10, **dadurch gekennzeichnet, daß** die Nutzpflanzen transgene Nutzpflanzen sind.

12. Verbindungen der Formel (II), 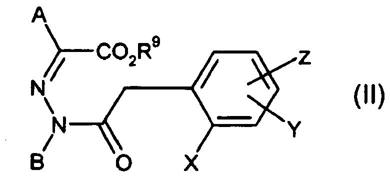 worin die Substituenten A, B, X, Y und Z wie unter Anspruch 1 oder 2 definiert sind, und R⁹ für (C₁-C₆)-Alkyl steht.

13. Verbindungen der Formel (IIa), 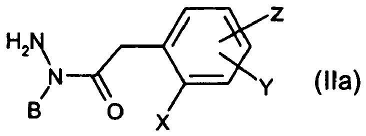 worin die Substituenten B, X, Y und Z wie unter Anspruch 1 oder 2 definiert sind.

14. Verwendung von 4-Phenylpyridazinonen der Formel (I) gemäß Anspruch 1 oder 2 als Insektizide.

## Claims

1. A 4-phenylpyridazinone of the formula (I) or salts thereof 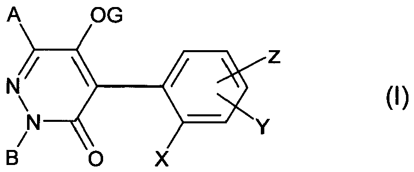 in which
A is (C₃-C₆)-cycloalkyl, (C₃-C₆)-cycloalkyl-(C₁-C₆)-alkyl, benzyl, halophenyl-(C₁-C₆)-alkyl or is (C₁-C₆)-alkyl which is substituted by n halogen atoms;
B is (C₃-C₆)-cycloalkyl, (C₃-C₆)-cycloalkyl-(C₁-C₆)-alkyl, benzyl, halophenyl-(C₁-C₆)-alkyl or is (C₁-C₆)-alkyl which is substituted by n halogen atoms;
m is 0, 1, 2 or 3;
n is 0, 1, 2 or 3, with the proviso that m and n are not both 0;
G is hydrogen, C(=O)R¹, C(=L)MR², SO₂R³, P(=L)R⁴R⁵, C(=L)NR⁶R⁷, or E;
E is Na⁺, K⁺, (Mg²⁺)_{1/2}, (Ca²⁺)_{1/2}, R¹³R¹⁴R¹⁵R¹⁶N⁺ or NH₄⁺;
R¹³, R¹⁴, R¹⁵ and R¹⁶ are independently of one another (C₁-C₆)-alkyl or benzyl;
L is oxygen;
M is oxygen;
R¹ is (C₁-C₆)-alkyl which is substituted by n halogen atoms or is (C₃-C₆)-cycloalkyl, phenyl or phenyl-(C₁-C₄)-alkyl, each of which is substituted by n radicals from the group consisting of halogen, (C₁-C₄)-alkyl and (C₁-C₄)-alkoxy;
R² is (C₁-C₆)-alkyl which is substituted by n halogen atoms or is (C₃-C₆)-cycloalkyl, phenyl or benzyl, each of which is substituted by n radicals from the group consisting of halogen, (C₁-C₄)-alkyl and (C₁-C₄)-alkoxy;
R³, R⁴ and R⁵ are each independently of one another (C₁-C₆)-alkyl which is substituted by n halogen atoms or are phenyl or benzyl which are substituted by n radicals from the group consisting of halogen, (C₁-C₄)-alkyl and (C₁-C₄)-alkoxy;
R⁶ and R⁷ are each independently of one another hydrogen, (C₁-C₆)-alkyl which is substituted by n halogen atoms or phenyl or benzyl which are substituted by n radicals from the group consisting of halogen, (C₁-C₄)-alkyl and (C₁-C₄)-alkoxy;
and
a)
X is hydrogen, methyl, ethyl or cyclopropyl;
Y is halogen, cyano, nitro, halo-(C₁-C₆)-alkyl, halo-(C₁-C₆)-alkoxy, (C₃-C₆)-cycloalkyl, (C₁-C₆)-alkoxy or phenyl which is substituted by n halogen atoms,
Z is hydrogen, halogen, halo-(C₁-C₆)-alkyl, halo-(C₁-C₆)-alkoxy, (C₃-C₆)-cycloalkyl or phenyl which is substituted by n halogen atoms,
or
b)
X is halogen, cyano, nitro, halo-(C₁-C₆)-alkyl, halo-(C₁-C₆)-alkoxy, (C₁-C₆)-alkoxy or (C₃-C₆)-cycloalkyl;
Y is hydrogen, halogen, cyano, nitro, (C₃-C₆)-cycloalkyl, is (C₁-C₆)-alkyl, (C₁-C₆)-alkoxy or phenyl, each of which is substituted by n halogen atoms;
Z is hydrogen, halogen, cyano, nitro, (C₃-C₆)-cycloalkyl or is (C₁-C₆)-alkyl, (C₁-C₆)-alkoxy or phenyl, each of which is substituted by n halogen atoms.

2. The 4-phenylpyridazinone as claimed in claim 1 in which
A is cyclopropyl, cyclopropylmethyl, benzyl, 2-chlorophenylmethyl, 3-chlorophenylmethyl or 4-chlorophenylmethyl;
B is hydrogen, 2,2-difluoroethyl, 2,2,2-trifluoroethyl, cyclopropyl, cyclopropylmethyl, benzyl, 2-chlorophenylmethyl, 3-chlorophenylmethyl or 4-chlorophenylmethyl;
G is hydrogen, C(=O)R¹, C(=L)MR², S02R3, P(=L)R⁴R⁵, C(=L)NR⁶R⁷, E;
E is Na⁺, K⁺, (Mg²⁺)_{1/2}, (Ca²⁺)_{1/2}, (CH₃)₄N⁺ or NH₄⁺;
L is oxygen;
M is oxygen;
R¹ is (C₁-C₆)-alkyl or (C₃-C6)-cycloalkyl;
R² is (C₁-C₆)-alkyl, (C₃-C₆)-cycloalkyl or benzyl;
R³, R⁴ and R⁵ are each independently of one another (C₁-C₆)-alkyl, phenyl or benzyl;
R⁶ and R⁷ are each independently of one another hydrogen, (C₁-C₆)-alkyl, phenyl or
benzyl; and
a)
X is hydrogen, methyl or ethyl;
Y is fluorine, bromine, chlorine, iodine, cyano, nitro, cyclopropyl, methoxy, ethoxy, trifluoromethyl or trifluoromethoxy, phenyl or halophenyl;
Z is hydrogen, fluorine, bromine, chlorine, iodine, methyl, ethyl, methoxy, ethoxy, trifluoromethyl or cyclopropyl;
or
b)
X is fluorine, bromine, chlorine, iodine, cyano, nitro, methoxy, ethoxy, trifluoromethyl, trifluoromethoxy or cyclopropyl;
Y is hydrogen, fluorine, bromine, chlorine, iodine, methyl, ethyl, methoxy, ethoxy, trifluoromethyl, trifluoromethoxy or cyclopropyl;
Z is hydrogen, fluorine, bromine, chlorine, iodine, methyl, ethyl, methoxy, ethoxy, trifluoromethyl, trifluoromethoxy, cyclopropyl, chlorophenyl or fluorophenyl.

3. A herbicidal composition which comprises an effective amount of at least one 4-phenylpyridazinone of the formula (I) as claimed in claim 1 or 2.

4. The herbicidal composition as claimed in claim 3 as a mixture with formulation auxiliaries.

5. The herbicidal composition as claimed in claim 3 or 4 which comprises at least one further pesticidally active compound from the group consiting of insecticides, acaricides, herbicides, fungicides, safeners and growth regulators.

6. The herbicidal composition as claimed in claim 5 which comprises a safener.

7. The herbicidal composition as claimed in claim 5 or 6 which comprises a further herbicide.

8. A method for controlling unwanted plants wherein an effective amount of at least one 4-phenylpyridazinone of the formula (I) as claimed in claim 1 or 2 or of a herbicidal composition as claimed in any of claims 3 to 7 is applied to the plants or to the site where the unwanted plants are growing.

9. The use of a 4-phenylpyridazinone of the formula (I) as claimed in claim 1 or 2 or of a herbicidal composition as claimed in any of claims 3 to 4 for controlling unwanted plants.

10. The use as claimed in claim 9 wherein the 4-phenylpyridazinone of the formula (I) is used for controlling unwanted plants in crops of useful plants.

11. The use as claimed in claim 10 wherein the useful plants are transgenic useful plants.

12. A compound of the formula (II), 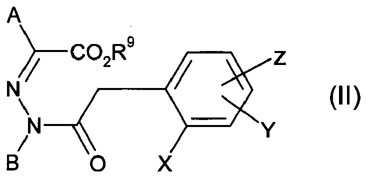 in which the substituents A, B, X, Y and Z are as defined in claim 1 or 2 and R⁹ is (C₁-C₆)-alkyl.

13. A compound of the formula (IIa) 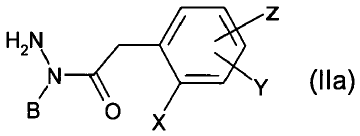 in which the substituents B, X, Y and Z are as defined in claim 1 or 2.

14. The use of a 4-phenylpyridazinone of the formula (I) as claimed in claim 1 or 2 as insecticides.

## Revendications

1. 4-Phénylpyridazinones de formule (I) ou leurs sels 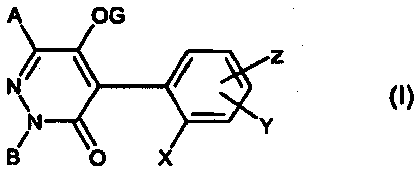 dans laquelle
A signifie cycloalkyle en (C₃-C₆), cycloalkyle en (C₃-C₆)-alkyle en (C₁-C₆), benzyle, halogénophényl-alkyle en (C₁-C₆) ou alkyle en (C₁-C₆) substitué par n atomes d'halogène ;
B signifie cycloalkyle en (C₃-C₆), cycloalkyle en (C₃-C₆)-alkyle en (C₁-C₆), benzyle, halogénophényl-alkyle en (C₁-C₆) ou alkyle en (C₁-C₆) substitué par n atomes d'halogèné ;
m signifie 0, 1, 2 ou 3 ;
n signifie 0, 1, 2 ou 3, à condition que m et n ne signifient pas simultanément 0 ;
G signifie hydrogène, C(=O) R¹, C (=L) MR², SO₂R³, P(=L)R⁴R⁵, C(=L)NR⁶R⁷ ou E ;
E Signifie Na⁺, K⁺, (Mg²⁺)_{1/2}, (Ca²⁺)_{1/2}, R¹³R¹⁴R¹⁵R¹⁶N⁺ ou NH₄+ ;
R¹³, R¹⁴, R¹⁵ et R¹⁶ signifient indépendamment les uns des autres alkyle en (C₁-C₆) ou benzyle ;
L signifie oxygène ;
M signifie oxygène ;
R¹ signifie alkyle en (C₁-C₆) substitué par n atomes d'halogène, ou cycloalkyle en (C₃-C₆), phényle ou phényl-alkyle en (C₁-C₄) chacun substitué par n radicaux du groupe constitué par halogène, alkyle en (C₁-C₄) et alcoxy en (C₁-C₄) ;
R² signifie alkyle en (C₁-C₆) substitué par n atomes d'halogène, ou cycloalkyle en (C₃-C₆), phényle ou benzyle chacun substitué par n radicaux du groupe constitué par halogène, alkyle en (C₁-C₄) et alcoxy en (C₁-C₄) ;
R³, R⁴ et R⁵ signifient chacun indépendamment les uns des autres alkyle en (C₁-C₆) substitué par n atomes d'halogène, ou phényle ou benzyle substitué par n radicaux du groupe constitué par halogène, alkyle en (C₁-C₄) et alcoxy en (C₁-C₄) ;
R⁶ et R⁷ signifient chacun indépendamment l'un de l'autre hydrogène, alkyle en (C₁-C₆) substitué par n atomes d'halogène, ou phényle ou benzyle substitué par n radicaux du groupe constitué par halogène, alkyle en (C₁-C₄) et alcoxy en (C₁-C₄) ;
et
a)
X signifie hydrogène, méthyle, éthyle ou cyclopropyle ;
Y signifie halogène, cyano, nitro, halogéno-alkyle en (C₁-C₆), halogéno-alcoxy en (C₁-C₆), cycloalkyle en (C₃-C₆) alcoxy en (C₁-C₆), ou phényle substitué par n atomes d'halogène,
Z signifie hydrogène, halogène, halogéno-alkyle en (C₁-C₆), halogéno-alcoxy en (C₁-C₆), cycloalkyle en (C₃-C₆), ou phényle substitué par n atomes d'halogène,
ou
b)
X signifie halogène, cyano, nitro, halogéno-alkyle en (C₁-C₆), halogéno-alcoxy en (C₁-C₆), alcoxy en (C₁-C₆) ou cycloalkyle en (C₃-C₆) ;
Y signifie hydrogène, halogène, cyano, nitro, cycloalkyle en (C₃-C₆) ; alkyle en (C₁-C₆), alcoxy en (C₁-C₆) ou phényle, chacun substitué par n atomes d'halogène ;
Z signifie hydrogène, halogène, cyano, nitro, cycloalkyle en (C₃-C₆) ; ou alkyle en (C₁-C₆), alcoxy en (C₁-C₆) ou phényle, chacun substitué par n atomes d'halogène.

2. 4-Phénylpyridazinones selon la revendication 1, dans lesquelles
A signifie cyclopropyle, cyclopropylméthyle, benzyle, 2-chlorophénylméthyle, 3-chlorophénylméthyle ou 4-chlorophénylméthyle ;
B signifie hydrogène, 2,2-difluoroéthyle, 2,2,2-trifluoroéthyle, cyclopropyle, cyclopropylméthyle, benzyle, 2-chloro-phénylméthyle, 3-chloro-phénylméthyle ou 4-chloro-phénylméthyle ;
G signifie hydrogène, C(=O)R¹, C(=L)MR², SO₂R³, P(=L)R⁴R⁵, C(=L)NR⁶R⁷, E ;
E signifie Na⁺, K⁺, (Mg²⁺)_{1/2}, (Ca²⁺)_{1/2}, (CH₃)₄N⁺ ou NH₄⁺ ;
L signifie oxygène ;
M signifie oxygène ;
R¹ signifie alkyle en (C₁-C₆) ou cycloalkyle en (C₃-C₆) ;
R² signifie alkyle en (C₁-C₆), cycloalkyle en (C₃-C₆) ou benzyle ;
R³, R⁴ et R⁵ signifient chacun indépendamment les uns des autres alkyle en (C₁-C₆), phényle ou benzyle ;
R⁶ et R⁷ signifient chacun indépendamment l'un de l'autre hydrogène, alkyle en (C₁-C₆), phényle ou benzyle ;
et
a)
X signifie hydrogène, méthyle ou éthyle ;
Y signifie fluor, brome, chlore, iode, cyano, nitro, cyclopropyle, méthoxy, éthoxy, trifluorométhyle ou trifluorométhoxy, phényle ou halogénophényle ;
Z signifie hydrogène, fluor, brome, chlore, iode, méthyle, éthyle, méthoxy, éthoxy, trifluorométhyle ou cyclopropyle ;
ou
b)
X signifie fluor, brome, chlore, iode, cyano, nitro, méthoxy, éthoxy, trifluorométhyle, trifluorométhoxy ou cyclopropyle ;
Y signifie hydrogène, fluor, brome, chlore, iode, méthyle, éthyle, méthoxy, éthoxy, trifluorométhyle, trifluorométhoxy ou cyclopropyle ;
Z signifie hydrogène, fluor, brome, chlore, iode, méthyle, éthyle, méthoxy, éthoxy, trifluorométhyle, trifluorométhoxy, cyclopropyle, chlorophényle ou fluorophényle.

3. Agents herbicides, **caractérisés par** une teneur herbicide efficace en au moins une 4-phénylpyridazinone de formule (I) selon la revendication 1 ou 2.

4. Agents herbicides selon la revendication 3, en mélange avec des adjuvants de formulation.

5. Agents herbicides selon la revendication 3 ou 4, contenant au moins un autre agent actif pesticide du groupe constitué par les insecticides, les acaricides, les herbicides, les fongicides, les agents protecteurs et les régulateurs de croissance.

6. Agents herbicides selon la revendication 5, contenant un agent protecteur.

7. Agents herbicides selon la revendication 5 ou 6, contenant un herbicide supplémentaire.

8. Procédé de lutte contre les plantes indésirables, **caractérisé en ce qu'**une quantité efficace d'au moins une 4-phénylpyridazinone de formule (I) selon la revendication 1 ou 2 ou d'un agent herbicide selon l'une quelconque des revendications 3 à 7 est appliqué sur les plantes ou à l'emplacement de la végétation indésirable.

9. Utilisation de 4-phénylpyridazinones de formule (I) selon la revendication 1 ou 2 ou d'agents herbicides selon l'une quelconque des revendications 3 à 4 pour la lutte contre les plantes indésirables.

10. Utilisation selon la revendication 9, **caractérisée en ce que** les 4-phénylpyridazinones de formule (I) sont utilisées pour lutter contre les plantes indésirables dans des cultures de plantes utiles.

11. Utilisation selon la revendication 10, **caractérisée en ce que** les plantes utiles sont des plantes utiles transgéniques.

12. Composés de formule (II) 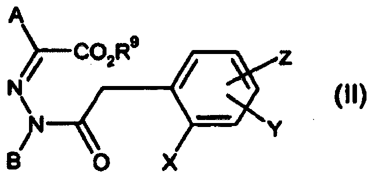 dans laquelle les substituants A, B, X, Y et Z sont tels que définis dans la revendication 1 ou 2, et R⁹ représente alkyle en (C₁-C₆) .

13. Composés de formule (IIa) 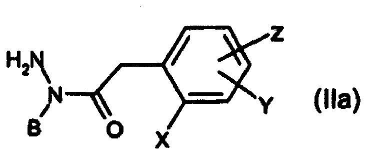 dans laquelle les substituants B, X, Y et Z sont tels que définis dans la revendication 1 ou 2.

14. Utilisation de 4-phénylpyridazinones de formule (I) selon la revendication 1 ou 2 en tant qu'insecticides.
